(19) Europäisches Patentamt
European Patent Office
Office européen des brevets

(11) Veröffentlichungsnummer: **0 310 551 B1**

(12) **EUROPÄISCHE PATENTSCHRIFT**

(45) Veröffentlichungstag der Patentschrift: **26.01.94**

(51) Int. Cl.[5]: **C07D 335/02**, C07D 495/10, G03C 7/00

(21) Anmeldenummer: **88810642.4**

(22) Anmeldetag: **21.09.88**

# (54) Phenolische Thianderivate.

(30) Priorität: **30.09.87 CH 3800/87**

(43) Veröffentlichungstag der Anmeldung:
**05.04.89 Patentblatt 89/14**

(45) Bekanntmachung des Hinweises auf die Patenterteilung:
**26.01.94 Patentblatt 94/04**

(84) Benannte Vertragsstaaten:
**BE DE FR GB IT NL**

(56) Entgegenhaltungen:
**EP-A- 0 031 456**
**EP-A- 0 103 540**

**ANGEWANDTE CHEMIE, INTERNATIONAL EDITION IN ENGLISH, Band 16, Nr. 3, März 1977, Seiten 196-197, Weinheim, DE; H.-J. GAIS: "Cyclic Dithiohemiacetals-Synthesis and properties"**

**JOURNAL OF ORGANIC CHEMISTRY, Band 52, Nr. 9, 1. Mai 1987, Seiten 1703-1710, Americam Chemical Society, Washington, US; H. MATSUYAMA et al.: "A regioselective synthesis of cyclopentenones from 4-thianone"**

(73) Patentinhaber: **CIBA-GEIGY AG**
**Klybeckstrasse 141**
**CH-4002 Basel(CH)**

(72) Erfinder: **Rody, Jean, Dr.**
**Rütiring 82**
**CH-4125 Riehen(CH)**
Erfinder: **Leppard, David G., Dr.**
**Route de Bourguillon 6**
**CH-1723 Marly(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue phenolische Derivate des Tetrahydrothiopyrans (Thian) und ihre Verwendung als Stabilisatoren für farbphotographische Aufzeichnungsmaterialien.

Es handelt sich hierbei um Verbindungen der Formel I oder II,

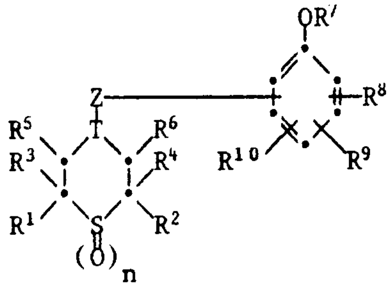

(I)

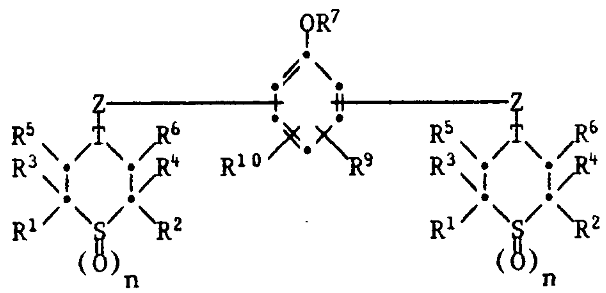

(II)

worin n 0, 1 oder 2 ist,

$R^1$ und $R^2$ unabhängig voneinander Wasserstoff oder Methyl bedeuten,

$R^3$ und $R^4$ unabhängig voneinander Wasserstoff, $C_1$-$C_4$-Alkyl, Phenyl, Thienyl oder durch 1 oder 2 $C_1$-$C_8$-Alkylgruppen, Cyclohexyl, Phenyl, $C_7$-$C_9$-Phenylalkyl, Hydroxyl, $C_1$-$C_{18}$-Alkoxy oder Halogen substituiertes Phenyl bedeuten,

$R^5$ und $R^6$ unabhängig voneinander Wasserstoff, $C_1$-$C_4$-Alkyl, Phenyl, -COO($C_1$-$C_{18}$-Alkyl), -CO-$CH_3$ oder -CO-Phenyl bedeuten,

$R^7$ Wasserstoff, $C_1$-$C_8$-Alkyl oder eine der Gruppen -CO-$R^{11}$, -CO-COO($C_1$-$C_4$-Alkyl), -$SO_2$-$R^{12}$, -CON($R^{13}$)($R^{14}$), -Si($R^{15}$)($R^{16}$)($R^{17}$) oder

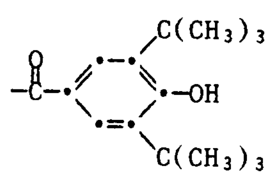

bedeutet,

$R^8$ und $R^9$ unabhängig voneinander Wasserstoff, $C_1$-$C_{12}$-Alkyl, $C_7$-$C_9$-Phenylalkyl, $C_5$-$C_8$-Cycloalkyl oder Phenyl bedeuten,

$R^{10}$ Wasserstoff, -$OR^7$ oder eine Gruppe der Formel III bedeutet,

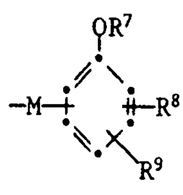

III

worin M eine direkte Bindung, -O-, -S-, -S-S-, -$CH_2$-, -CH($C_1$-$C_8$-Alkyl)- oder -$C(CH_3)_2$- bedeutet,

$R^{11}$ $C_1$-$C_{20}$-Alkyl, $C_3$-$C_{20}$-Alkenyl, $C_5$-$C_{12}$-Cycloalkyl, $C_7$-$C_{13}$-Phenylalkyl oder $C_6$-$C_{10}$-Aryl bedeutet,

$R^{12}$ $C_1$-$C_{12}$-Alkyl, $C_6$-$C_{10}$-Aryl oder $C_7$-$C_{24}$-Alkylaryl bedeutet,
$R^{13}$ Wasserstoff, $C_1$-$C_{12}$-Alkyl oder Cyclohexyl bedeutet,
$R^{14}$ $C_1$-$C_{12}$-Alkyl, $C_6$-$C_{10}$-Aryl, durch $C_1$-$C_{12}$-Alkyl substituiertes $C_6$-$C_{10}$-Aryl oder Cyclohexyl bedeutet, oder $R^{13}$ und $R^{14}$ zusammen mit dem N-Atom einen 5- oder 6-gliedrigen gesättigten heterocyclischen Ring bilden,
$R^{15}$, $R^{16}$ und $R^{17}$ unabhängig voneinander $C_1$-$C_{12}$-Alkyl, $C_3$-$C_{12}$-Alkenyl, Phenyl, Cyclohexyl oder Benzyl bedeuten.
T eine dreiwertige Gruppe ist, die den Ring zu einem Thianring ergänzt und eine der folgenden Gruppen ist:
$>$CH-O-, $>$C = N-NH-,

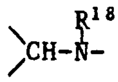 , 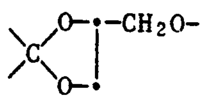 oder 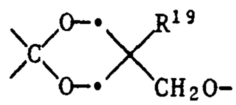 ,

worin $R^{18}$ Wasserstoff, $C_1$-$C_{12}$-Alkyl, Benzyl, Cyclohexyl oder Phenyl ist und $R^{19}$ Wasserstoff oder $C_1$-$C_4$-Alkyl ist,
Z ein zweiwertiges Bindeglied zwischen T und dem Phenolrest ist und eine der folgenden Gruppen ist:

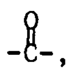 , 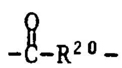 , 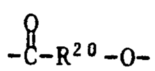 oder 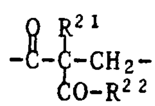 ,

deren Carbonylgruppe an T gebunden ist und worin
$R^{20}$ $C_1$-$C_{14}$-Alkylen bedeutet,
$R^{21}$ Wasserstoff, $C_1$-$C_{12}$-Alkyl, Phenyl, $C_7$-$C_9$-Phenylalkyl oder eine Gruppe der Formel IV bedeutet,

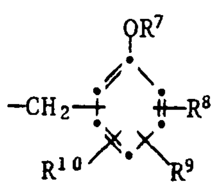

IV

und $R^{22}$ eine Gruppe -O($C_1$-$C_4$-Alkyl) oder eine Gruppe der Formel V

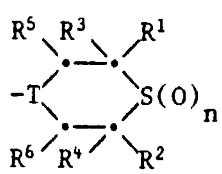

V

bedeutet.

Soweit es sich bei diesen Substituenten um Alkyl oder Alkylen handelt, können diese Gruppen unverzweigt oder verzweigt sein. Wenn $R^{13}$ und $R^{14}$ zusammen mit dem N-Atom einen gesättigten heterocyclischen Ring bilden, können sie zusätzlich noch ein O-, N- oder S-Atom enthalten. Beispiele sind ein Pyrrolidin-, Piperidin-, Morpholin- oder Piperazinring, der gegebenenfalls durch ein oder zwei $C_1$-$C_4$-Alkylgruppen substituiert sein kann.

Bevorzugt sind Verbindungen der Formel I oder II, worin n 0 oder 2 ist, $R^1$ und $R^2$ Wasserstoff oder Methyl bedeuten, $R^3$ und $R^4$ unabhängig voneinander Methyl, Phenyl, Thienyl oder durch ein oder zwei $C_1$-$C_4$-Alkylgruppen, Cyclohexyl, Hydroxyl, $C_1$-$C_4$-Alkoxy oder Chlor substituiertes Phenyl bedeuten,
$R^5$ und $R^6$ unabhängig voneinander Wasserstoff, -COO($C_1$-$C_4$-Alkyl) oder $-COCH_3$ bedeuten,
$R^7$ Wasserstoff, eine Gruppe -CO-$R^{11}$, -CO-COO($C_1$-$C_4$-Alkyl), $-Si(CH_3)_3$ oder

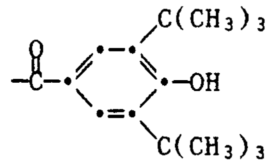

bedeutet,
$R^8$ und $R^9$ unabhängig voneinander $C_1$-$C_8$-Alkyl, $C_7$-$C_9$-Phenylalkyl, Cyclohexyl oder Phenyl bedeuten,
$R^{10}$ Wasserstoff, -$OR^7$ oder eine Gruppe der Formel III bedeutet,
worin M -S-, -$CH_2$-, -CH($C_1$-$C_4$-Alkyl) oder -$C(CH_3)_2$- ist,
$R^{11}$ $C_1$-$C_{12}$-Alkyl oder Phenyl bedeutet,
T eine der folgenden dreiwertigen Gruppen ist:
>CH-O-, >C=N-NH-,

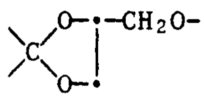 oder 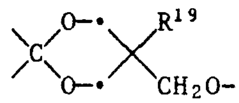 ,

worin $R^{19}$ $C_1$-$C_4$-Alkyl bedeutet,
und Z eine der folgenden zweiwertigen Gruppen ist:

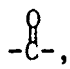, 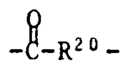, 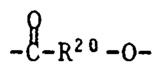 oder 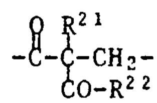 ,

deren Carbonylgruppe an T gebunden ist und worin
$R^{20}$ $C_1$-$C_{14}$-Alkylen ist,
$R^{21}$ $C_1$-$C_8$-Alkyl, Benzyl oder eine Gruppe der Formel IV ist und
$R^{22}$ eine Gruppe -O($C_1$-$C_4$-Alkyl) oder eine Gruppe der Formel V ist.

Besonders bevorzugt sind Verbindungen der Formel I und II, worin
n 0 oder 2 ist,
$R^1$ und $R^2$ Wasserstoff oder Methyl bedeuten,
$R^3$ und $R^4$ Methyl, Phenyl, Thienyl oder durch $C_1$-$C_4$-Alkyl, $C_1$-$C_4$-Alkoxy, Hydroxyl oder Chlor substituiertes Phenyl bedeuten,
$R^5$ und $R^6$ Wasserstoff sind,
$R^7$ Wasserstoff oder eine Gruppe

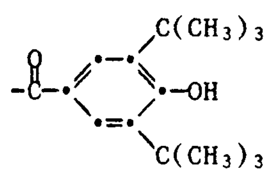

bedeutet,
$R^8$ und $R^9$ unabhängig voneinander Wasserstoff, $C_1$-$C_4$-Alkyl, Cyclohexyl oder Phenyl bedeuten,
$R^{10}$ Wasserstoff ist,
T eine der folgenden dreiwertigen Gruppen ist:
>CH-O-,

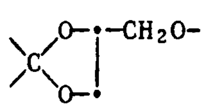 oder 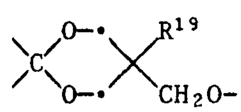 ,

worin $R^{19}$ $C_1$-$C_4$-Alkyl ist,
und Z eine der folgenden zweiwertigen Gruppen ist:

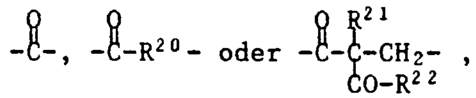

deren Carbonylgruppe an T gebunden ist und worin
$R^{20}$ $C_1$-$C_8$-Alkylen bedeutet,
$R^{21}$ $C_1$-$C_8$-Alkyl, Benzyl oder eine Gruppe der Formel IV ist und
$R^{22}$ -O($C_1$-$C_4$-Alkyl) oder eine Gruppe der Formel V ist.

Bevorzugt sind Verbindungen der Formel I und II, worin $R^1$, $R^2$, $R^5$ und $R^6$ Wasserstoff sind.

Der Schwefel im Thianring kann als Sulfid-, Sulfoxid- oder Sulfon-Schwefel vorliegen, je nachdem, ob n null, 1 oder 2 ist. Bevorzugt ist n null oder 2, insbesondere null.

Gegenüber den Verbindungen der Formel II sind die Verbindungen der Formel I bevorzugt.

Beispiele für Verbindungen der Formel I sind:

1) 4-(3,5-Di-tert.butyl-4-hydroxybenzoyloxy)-thian
2) 4-[3-(3,5-Di-tert.butyl-4-hydroxyphenyl)-propionyloxy]-thian
3) 4-(3,5-Di-tert.butyl-4-hydroxybenzoyloxy)-2,2,6,6-tetramethylthian
4) 4-[3-(3,5-Di-tert.butyl-4-hydroxyphenyl)-propionyloxy]-2,2,6,6-tetramethylthian
5) 4-[3-(3,5-Di-tert.butyl-4-hydroxyphenyl)-propionyloxy]-1-oxo-2,2,6,6-tetramethylthian
6) 4-[3-(3,5-Di-tert.butyl-4-hydroxyphenyl)-propionyloxy]-1,1-dioxo-2,2,6,6-tetramethylthian
7) 4-[5,5-Dimethyl-5-(2-hydroxy-5-methoxyphenyl)-valerianyloxy]-2,2,6,6-tetramethylthian
8) 4-[α,α-Bis(3,5-di-tert.butyl-4-hydroxybenzyl)-α-methoxycarbonylacetoxy]-2,2,6,6-tetramethylthian
9) 4-(3,5-Di-tert.butyl-4-hydroxybenzoyloxy)-2,6-diphenylthian
10) 4-[3-(3,5-Di-tert.butyl-4-hydroxyphenyl)-propionyloxy]-2,6-diphenylthian
11) N-[3-(3,5-Di-tert.butyl-4-hydroxyphenyl)-propionyloxy]-2,6-diphenylthian-4-hydrazon
12) 4-[3-(3,5-Di-tert.butyl-4-hydroxyphenyl)-propionyloxy]-1,1-dioxo-2,6-diphenylthian
13) 4-[3-(3-Methyl-4-hydroxy-5-tert.butylphenyl)-propionyloxy]-1,1-dioxo-2,6-diphenylthian
14) N-[3-(3-Methyl-4-hydroxyphenyl-5-tert.butylphenyl)-propionyloxy]-2,6-diphenylthian-4-hydrazon
15) 1,4-Dioxa-3-(3,5-di-tert.butyl-4-hydroxybenzoyloxymethyl)-7,9-diphenyl-8-tetra[4.5]spirodecan
16) 1,4-Dioxa-3-[β-(3,5-di-tert.butyl-4-hydroxyphenyl)-propionyloxymethyl]-7,9-diphenyl-8-thia[4.5]-spirodecan
17) 1,4-Dioxa-3-[β-(3-methyl-4-hydroxy-5-tert.butylphenyl)-propionyloxymethyl]-7,9-diphenyl-8-thia[4.5]-spirodecan
18) 1,5-Dioxa-3-ethyl-(3,5-di-tert.butyl-4-hydroxybenzoyloxymethyl)-8,10-diphenyl-9-thia[5.5]spiroundecan
19) 1,5-Dioxa-3-ethyl-3-[β-(3,5-di-tert.butyl-4-hydroxyphenyl)-propionyloxymethyl]-8,10-diphenyl-9-thia-[5.5]spiroundecan
20) 1,5-Dioxa-3-ethyl-3-[β-(3-methyl-4-hydroxy-5-tert.butylphenyl)-propionyloxymethyl]-8,10-diphenyl-9-thia[5.5]spiroundecan
21) 1,5-Dioxa-3-ethyl-3-[β-(3,5-di-tert.butyl-4-hydroxyphenyl)-propionyloxymethyl]-8,10-diphenyl-9-thia-9,9-dioxo[5.5]spiroundecan
22) 1,5-Dioxa-3-ethyl-3-[β-(3-methyl-4-hydroxy-5-tert.butylphenyl)-propionyloxymethyl]-8,10-diphenyl-9-thia-9,9-dioxo[5.5]spiroundecan
23) 1,5-Dioxa-3-ethyl-3-[α-(3,5-di-tert.butyl-4-hydroxyphenoxy)-acetyloxymethyl]-8,10-diphenyl-9-thia[5.5]-undecan
24) 1,5-Dioxa-3-ethyl-3-[β-(3-methyl-4-methyloxalyloxy-5-tert.butylphenyl)-propionyloxymethyl]-8,10-diphenyl-9-thia[5.5]undecan
25) 1,5-Dioxa-3-ethyl-3-[β-(3-methyl-4-trimethylsiloxy-5-tert.butyl)-propionyloxymethyl]-8,10-diphenyl-9-thia[5.5]undecan
26) 1,5-Dioxa-3-ethyl-3-[5,5-dimethyl-5-(2-hydroxy-5-methoxyphenyl)-valerianyloxymethyl]-8,10-diphenyl-9-thia[5.5]undecan
27) 1,5-Dioxa-3-ethyl-3-[2-(3-tert.butyl-4-hydroxyphenyl)-tetradecanoyloxymethyl]-8,10-diphenyl-9-thia-[5.5]undecan
28) 1,5-Dioxa-3-ethyl-3-[5,5-dimethyl-5-(2-hydroxy-3,5-dimethylphenyl)-valerianyloxymethyl]-8,10-diphenyl-9-thia[5.5]undecan

29) 1,5-Dioxa-3-ethyl-3-[5,5-dimethyl-5-(3-methyl-4-hydroxyphenyl)-valerianyloxymethyl]-8,10-diphenyl-9-thia[5.5]undecan
30) 1,5-Dioxa-3-ethyl-3-[5,5-dimethyl-5-(2-hydroxy-5-tert.butylphenyl)-valerianyloxymethyl]-8,10-diphenyl-9-thia[5.5]undecan
31) 1,5-Dioxa-3-ethyl-3-[5,5-dimethyl-5-(3,5-dimethyl-4-hydroxyphenyl)-valerianyloxymethyl]-8,10-diphenyl-9-thia[5.5]undecan
32) 1,5-Dioxa-3-ethyl-3-[5,5-dimethyl-5-(2-methyloxalyloxy-3,5-dimethyl)-valerianyloxymethyl]-8,10-diphenyl-9-thia[5.5]undecan
33) 1,5-Dioxa-3-ethyl-3-[5,5-dimethyl-5-(3,5-di-tert.butyl-4-hydroxyphenyl)-valerianyloxymethyl]-8,10-diphenyl-9-thia[5.5]undecan
34) 4-(3,5-Di-tert.butyl-4-hydroxybenzoyloxy)-2,6-di(4-chlorphenyl)-thian
35) 4-[3-(3,5-Di-tert.butyl-4-hydroxyphenyl)-propionyloxy]-2,6-di(4-chlorphenyl)-thian
36) 4-[3-(3-Methyl-4-hydroxy-5-tert.butylphenyl)-propionyloxy]-2,6-di(4-chlorphenyl)-thian
37) 4-[3-(3,5-Di-tert.butyl-4-hydroxyphenyl)-propionyloxy]-2,6-di(4-methoxyphenyl)-thian
38) 1,5-Dioxa-3-ethyl-3-(3,5-di-tert.butyl-4-hydroxybenzoyloxymethyl-8,10-di(4-chlorphenyl)-9-thia[5.5]-undecan
39) 1,5-Dioxa-3-ethyl-3-[3-(3,5-di-tert.butyl-4-hydroxyphenyl)-propionyloxymethyl]-8,10-di(4-chlorphenyl)-9-thia[5.5]undecan
40) 1,5-Dioxa-3-ethyl-3-[3-(3-methyl-4-hydroxy-5-tert.butylphenyl)-propionyloxymethyl]-8,10-di-(4-chlorphenyl)-9-thia[5.5]undecan
41) 1,5-Dioxa-3-ethyl-3-[3-(3-methyl-4-hydroxy-5-tert.butylphenyl)-propionyloxymethyl]-8,10-di(3-methyl-4-hydroxy-5-tert.butylphenyl)-9-thia[5.5]undecan
42) 4-(3,5-Di-tert.butyl-4-hydroxybenzoyloxy)-2,6-di(2-thienyl)-thian
43) 4-[3-(3,5-Di-tert.butyl-4-hydroxyphenyl)-propionyloxy]-2,6-di(2-thienyl)-thian
44) 4-[3-(3-Methyl-4-hydroxy-5-tert.butylphenyl)-propionyloxy]-2,6-di(2-thienyl)-thian
45) 1,5-Dioxa-3-ethyl-3-[β-(3-tert.butyl-4-hydroxyphenyl)-propionyloxymethyl]-8,10-diphenyl-9-thia[5.5]-spiroundecan
46) 1,5-Dioxa-3-ethyl-3-[β-(3-methyl-5-tert.butyl-4-[methyloxalyloxy]-phenyl)-propionyloxymethyl]-8,10-diphenyl-9-thia[5.5]spiro undecan
47) 1,5-Dioxa-3-ethyl-3-[β-(3-methyl-5-tert.butyl-4-[trimethylsiloxy]-phenyl)-propionyloxymethyl]-8,10-diphenyl-9-thia[5.5]spiroundecan
48) N-[β-(3-Methyl-4-hydroxy-5-tert.butylphenyl)-propionylamido]-2,6-diphenyl-4-iminothian
49) N-[β-(3,5-Di-tert.butyl-4-hydroxyphenyl)-propionylamido]-2,6-diphenyl-4-iminothian
50) Bis-(3,5-di-tert.butyl-4-hydroxybenzyl)-malonsäure-monomethylester-mono(2,2,6,6-tetramethylthian-4-yl)-ester
51) Bis-(3,5-di-tert.butyl-4-hydroxybenzyl)malonsäure-di(2,2,6,6-tetramethylthian-4-yl)-ester
52) Die Verbindung der Formel

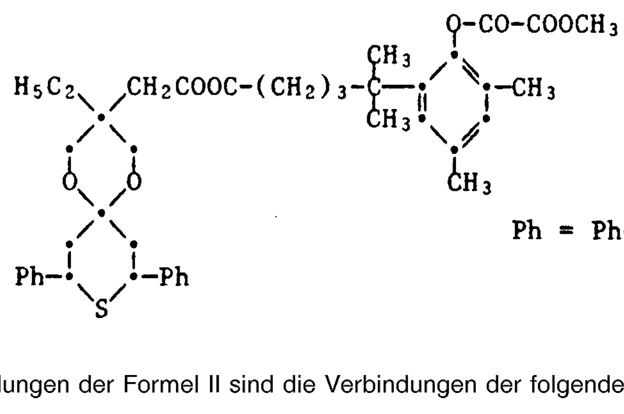

Ph = Phenyl

Beispiele für Verbindungen der Formel II sind die Verbindungen der folgenden Formeln:

53)

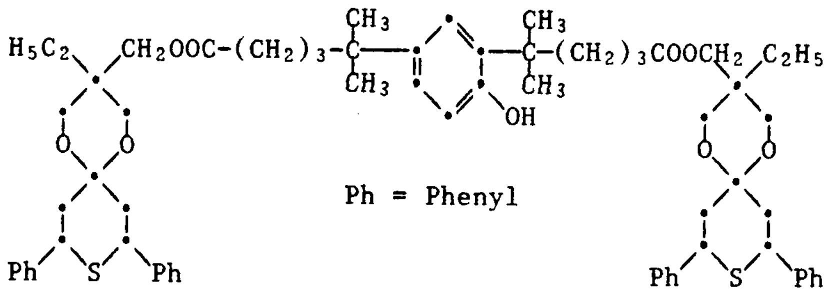

Ph = Phenyl

54)

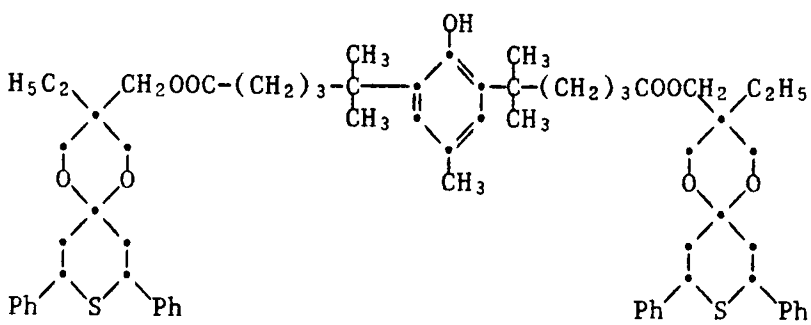

55)

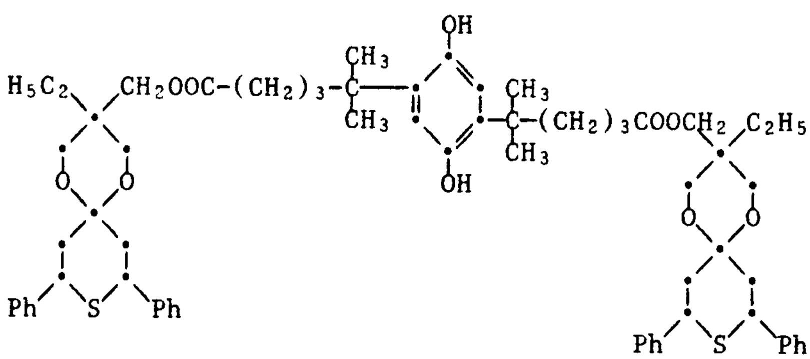

56)

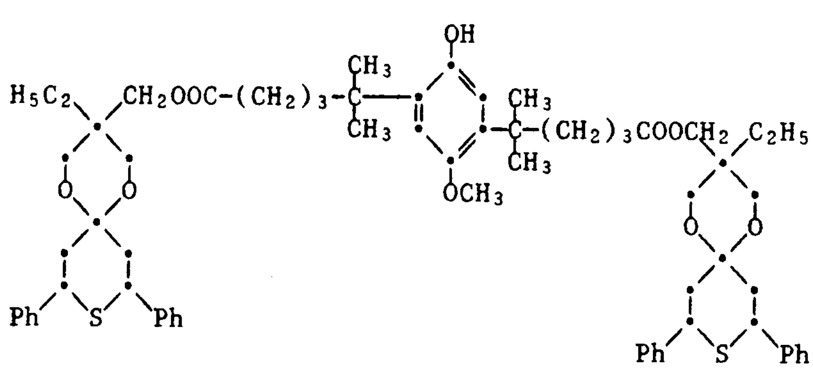

57)

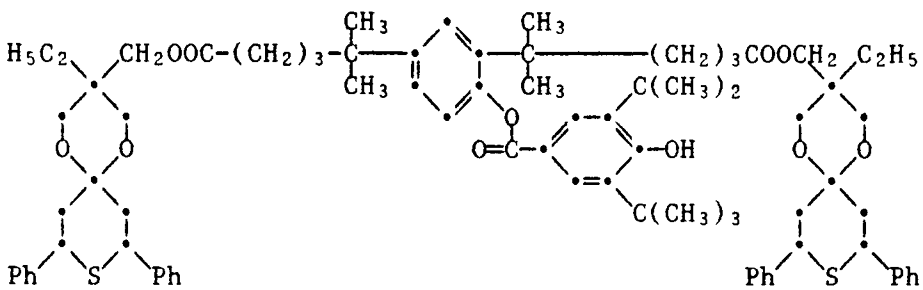

58)

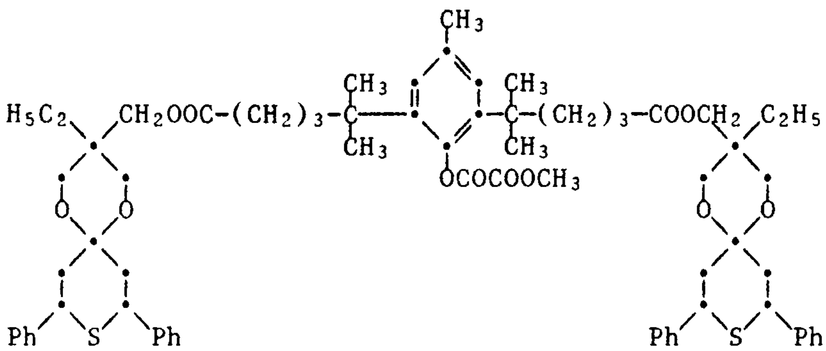

59)

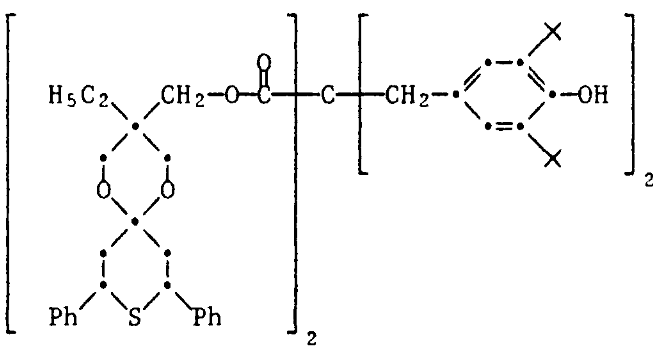

Die Verbindungen der Formel I oder II können durch Umsetzung einer phenolischen Carbonsäure oder deren Derivat VI mit einem OH- oder NH-funktionellen Thianderivat VII hergestellt werden:

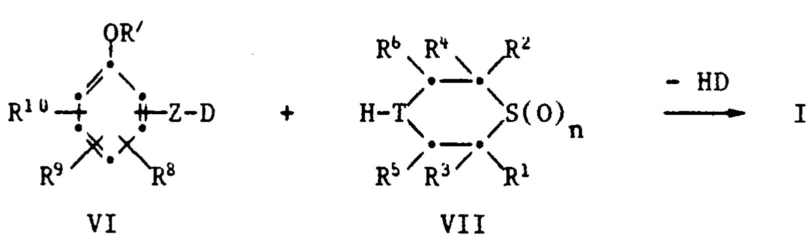

D = OH, O($C_1$-$C_4$-Alkyl) oder Cl

Analog erfolgt die Herstellung von Verbindungen der Formel II durch Reaktion einer phenolischen Dicarbonsäure oder deren Derivat VIII mit 2 Aequivalenten VII:

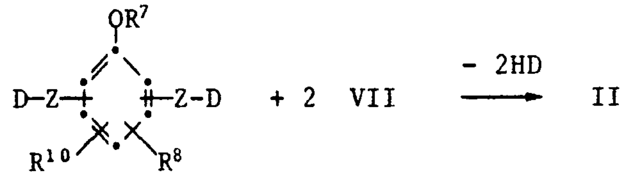


VIII

Ist VII ein OH-funktionelles Thianderivat, so erfolgt die Reaktion unter den üblichen Bedingungen einer Veresterung (D = OH), einer Umesterung (D = O-Alkyl) oder einer O-Acylierung (D = Cl). Die Veresterung erfolgt vorzugsweise unter saurer Katalyse, die Umesterung unter basischer Katalyse. Die O-Acylierung mittels des Carbonsäurechlorides erfolgt vorzugsweise in Gegenwart äquivalenter Mengen einer Base.

Die phenolischen Carbonsäuren und ihre Derivate der Formel VI und VIII sind bekannte Verbindungen oder können nach an sich bekannten Methoden hergestellt werden.

Die funktionellen Thiane der Formel VII sind z.T. bekannte Verbindungen oder können in Analogie zu bekannten Verfahren hergestellt werden. So können Verbindungen der Formel VII, worin >T-H eine Gruppe >CH-OH ist, durch Reduktion der entsprechenden Ketone mit Boranaten gemäss J. Klein, H. Stollar/Tetrahedron 30, 2541 (1974) oder K. Ramalingam et al./J. Org. Chem. 44, 477 (1979) hergestellt werden. Verbindungen der Formel VII, worin >T-H eine Gruppe >CH-$NH_2$ ist, können durch Reduktion der Oxime >C=NOH mit $LiAlH_4$ gemäss P.K. Subramanian et al./J. Org. Chem. 46, 4376 (1981) hergestellt werden. Verbindungen der Formel VII, worin >T-H eine Gruppe >C=N-$NH_2$ ist, werden durch Umsetzung der entsprechenden Thianone mit Hydrazin erhalten. Verbindungen der Formel VII, worin >T-H eine Gruppe

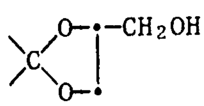

oder

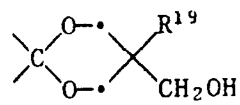

ist, können durch Ketalisierung der entsprechenden Thianone mit Glycerin oder mit einem Polyol der Formel $R^{19}$-$C(CH_2OH)_3$ erhalten werden.

Die cyclischen Sulfoxide und Sulfone der Formel I oder II, worin n 1 oder 2 ist, können aus den Thianderivaten mit n = 0 durch Oxidation gewonnen werden, wie dies z.B. beschrieben ist von J. Klein, H. Stollar/Tetrahedron 30, 2541 (1974) und P.K. Subramanian et. al./J. Org. Chem. 46, 4376 (1981).

Verbindungen der Formel I, in denen T eine Gruppe >C=N-NH- ist, können auch durch Reaktion eines Thianons IX mit einem Hydrazid einer phenolischen Carbonsäure X hergestellt werden:

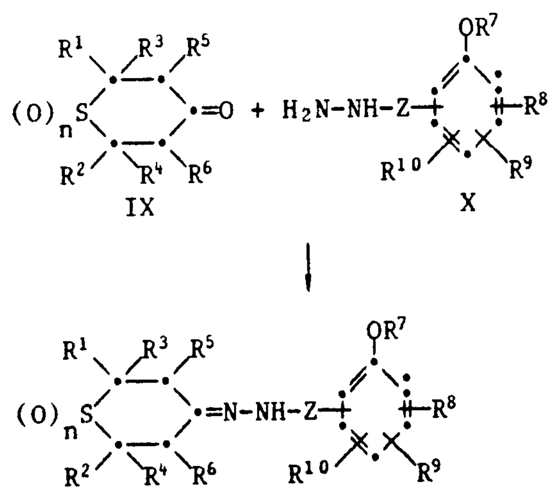

IX

X

Die Verbindungen der Formel I oder II können als Stabilisatoren für farbphotographische Aufzeichnungsmaterialien verwendet werden. Diese Materialien sind vorwiegend Papiere oder Filme, die drei lichtempfindliche Schichten enthalten, die Gelbschicht, die Magentaschicht und die Cyanschicht. Diese Schichten sind Gelatineschichten, die mindestens ein Silberhalogenid und einen Farbstoffkuppler enthalten und noch weitere Zusätze enthalten können. Die Verbindungen der Formel I oder II werden einer solchen Gelatineschicht zugesetzt. Hierzu löst man sie in einem organischen Lösungsmittel bzw. Lösungsmittelgemisch und emulgiert die Lösung in einer Gelatinelösung, die dann der photographischen Gelatineschicht bei deren Bereitung zugesetzt wird. Als Lösungsmittel verwendet man bevorzugt ein Gemisch eines niedrig-siedenden und eines hochsiedenden Lösungsmittels und entfernt das niedrig-siedende Lösungsmittel während der Emulgierung.

Das Dispergieren der Stabilisatorlösung in der Gelatinelösung kann z.B. in einer Kolloidmühle oder in einem Homogenisator oder mit Hilfe von Ultraschall geschehen. Hierbei können auch oberflächenaktive Mittel (Emulgatoren) zugesetzt werden. Eine feine Dispergierung ist Voraussetzung für die homogene Verteilung der Stabilisatoren in der photographischen Schicht.

Die Verbindungen der Formel I oder II stabilisieren sowohl die Farbkuppler als auch die nach Belichtung und Entwicklung entstandenen Photofarbstoffe gegen Lichteinwirkung. Sie verhindern oder verzögern das Ausbleichen oder die Farbänderung der Photofarbstoffe durch Lichteinwirkung. Sie reagieren nicht mit den üblichen Farbstoffkupplern und beeinträchtigen den photographischen Prozess der Farbbildung nicht.

Es wurden bereits phenolische Verbindungen als Stabilisatoren für farbphotographische Materialien vorgeschlagen, so z.B. in der EP-A-82817, EP-A-103540, US-A-3 935 016 oder EP-A-113 124. Solche Stabilisatoren haben zu einer erheblichen Steigerung der Lichtechtheit von Farbphotographien geführt, aber man ist weiterhin an einer Verbesserung der Stabilisierung interessiert.

Man setzt die Stabilisatoren der Formel I oder II pro Farbschicht zweckmässig in einer Menge von bis zu 1 $g/m^2$, vorzugsweise 10 bis 300 $mg/m^2$, zu. Der Zusatz kann zu einer oder zwei oder allen drei Farbsilberschichten erfolgen. Von besonderer Bedeutung ist der Zusatz zur Gelbschicht. In den Schichten befindet sich das sensibilisierte Silberhalogenid und der jeweilige Farbkuppler. Ausserdem können die Schichten weitere Stabilisatoren und/oder sonstige Zusätze enthalten.

Die Gelbkuppler sind vorzugsweise Verbindungen der Formel XI,

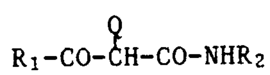 XI

worin $R_1$ Alkyl oder Aryl ist, $R_2$ Aryl ist und Q Wasserstoff oder eine Gruppe ist, die durch Reaktion mit dem oxidierten Entwickler abgespalten werden kann.

Eine Gruppe von Gelbkupplern sind solche Verbindungen der Formel XI, in denen $R_1$ tert-Butyl ist und $R_2$ eine Gruppe der Formel

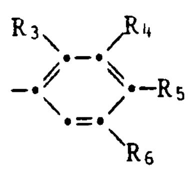

ist, worin $R_3$ Wasserstoff, Halogen, Alkyl oder Alkoxy bedeutet und $R_4$, $R_5$ und $R_6$ Wasserstoff, Halogen, Alkyl, Alkenyl, Alkoxy, Aryl, Carboxy, Alkoxycarbonyl, eine Carbamoylgruppe, eine Sulfon- oder Sulfamoylgruppe, eine Alkylsulfonamidogruppe, Acylaminogruppe, Ureidogruppe oder Aminogruppe bedeuten.

Vorzugsweise sind $R_3$ Chlor, $R_4$ und $R_5$ Wasserstoff und $R_6$ eine Acylaminogruppe. Hierzu gehören auch die Verbindungen der Formel

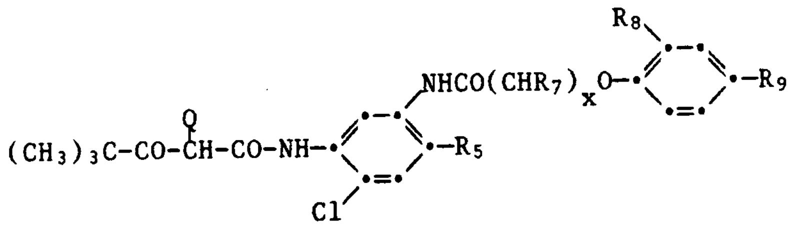

,

worin x 0-4 ist, $R_7$ Wasserstoff oder Alkyl bedeutet, und $R_8$ und $R_9$ Alkyl sind.

Eine andere Gruppe von Gelbkupplern entspricht der Formel XII

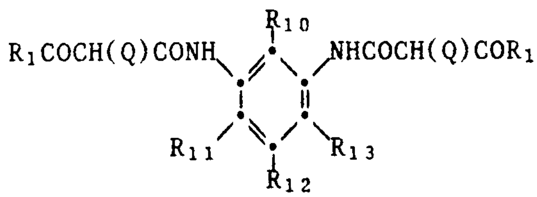

XII ,

worin $R_{10}$ Wasserstoff, Halogen oder Alkoxy ist,
$R_{11}$, $R_{12}$ und $R_{13}$ Wasserstoff, Halogen, Alkyl, Alkenyl, Alkoxy, Aryl, Carboxyl, Alkoxycarbonyl, eine Carbamoylgruppe, eine Sulfongruppe, Sulfamoylgruppe, Sulfonamidogruppe, Acylaminogruppe, Ureidogruppe oder Aminogruppe bedeuten und $R_1$ und Q die oben angegebene Bedeutung haben.

Dazu gehören Verbindungen der Formel XII, in denen $R_1$ tert.Butyl ist, $R_{10}$ Chlor ist, $R_{11}$ und $R_{13}$ Wasserstoff sind und $R_{12}$ Alkoxycarbonyl ist.

In den Verbindungen der Formel XI und XII kann die Abgangsgruppe Q Wasserstoff sein oder sie ist eine heterocyclische Gruppe

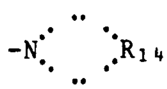

,

worin $R_{14}$ eine organische zweiwertige Gruppe ist, die den Ring zu einem 4-7-gliedrigen Ring ergänzt oder Q ist eine Gruppe $-OR_{15}$, worin $R_{15}$ Alkyl, Aryl, Acyl oder ein heterocyclischer Rest ist.

Typische Beispiele für gebräuchliche Gelbkuppler sind die Verbindungen der folgenden Formeln:

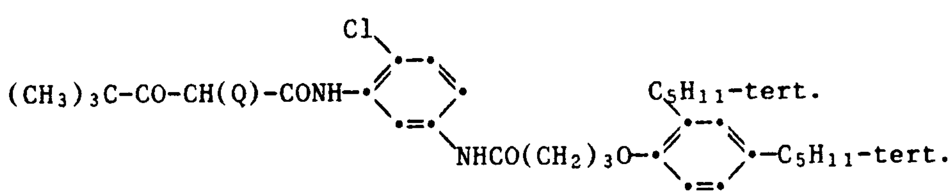

a) Q = 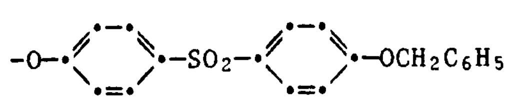

b) Q = 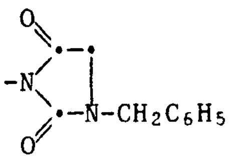

c) Q = 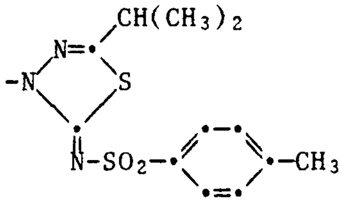

d) Q = 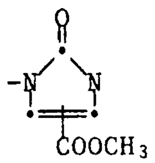

e) Q = 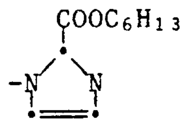

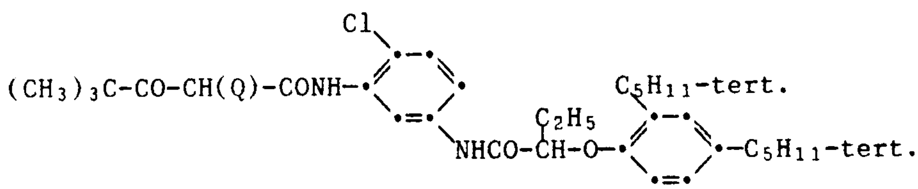

f) Q = 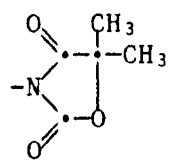

g) Q = 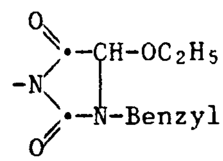

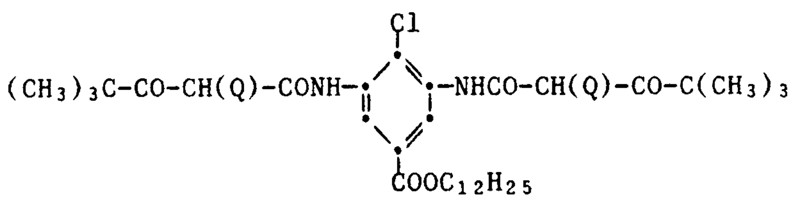

h) Q = 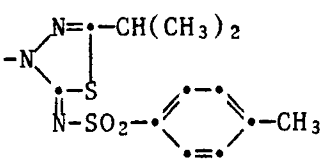

Weitere Beispiele für Gelbkuppler sind zu finden in den US-A 2,407,210, 2,778,658, 2,875,057, 2,908,513, 2,908,573, 3,227,155, 3,227,550, 2,253,924, 3,265,506, 3,227,155, 3,408,194, 3,341,331, 3,369,895, 3,384,657, 3,415,652, 3,447,928, 3,551,155, 3,582,322, 3,725,072, 3,891,445, 3,933,501,4,115,121, 4,401,752, 4,022,620, in den DE-A 1,547,868, 2,057,941, 2,162,899, 2,163,813, 2,213,461, 2,219,917, 2,261,361, 2,261,362, 2,263,875, 2,329,587, 2,414,006, 2,422,812 und in den GB-A 1,425,020 und 1,077,874.

Die Gelbkuppler werden üblicherweise in einer Menge von 0,05-2 Mol und vorzugsweise 0,1-1 Mol pro Mol Silberhalogenid verwendet.

Magentakuppler können z.B. einfache 1-Aryl-5-pyrazolone sein oder mit 5-gliederigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel XIII,

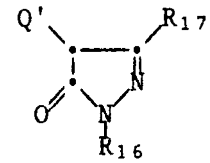

XIII

wie sie in der Britischen Patentschrift 2,003,473 beschrieben sind. Darin ist $R_{16}$ Wasserstoff, Alkyl, Aryl, Alkenyl oder eine heterocyclische Gruppe. $R_{17}$ ist Wasserstoff, Alkyl, Aryl, eine heterocyclische Gruppe, eine Estergruppe, Alkoxygruppe, Alkylthiogruppe Carboxylgruppe, Arylaminogruppe, Acylaminogruppe, (Thio)-harnstoffgruppe, (Thio)-carbamoylgruppe, Guanidinogruppe oder Sulfonamidogruppe.

Bevorzugt ist $R_{17}$ eine Gruppe

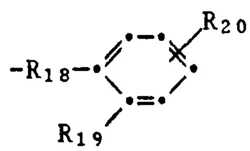

worin $R_{18}$ Imino, Acylamino oder Ureido ist, $R_{19}$ Wasserstoff, Halogen, Alkyl oder Alkoxy ist, $R_{20}$ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Typische Beispiele für diesen Typ von Magentakupplern sind Verbindungen der Formel

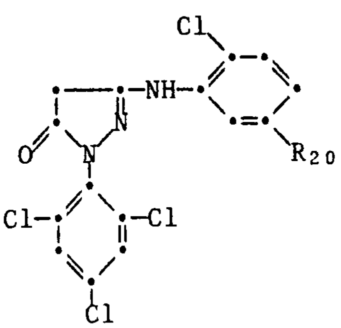

,

worin $R_{20}$ die oben genannten Bedeutungen hat.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866, 3,933,500.

Wenn Q' in Formel XIII nicht Wasserstoff ist sondern eine Gruppe, die bei der Reaktion mit dem oxidierten Entwickler eliminert wird, so handelt es sich um einen diäquivalenten Magentakuppler. Q' kann in diesem Fall z.B. Halogen oder eine über O, S oder N an den Pyrazolring gebundene Gruppe sein. Solche diäquivalente Kuppler ergeben eine höhere Farbdichte und sind reaktiver gegenüber dem oxidierten Entwickler als die entsprechenden tetraäquivalenten Magentakuppler.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, im EP-A-133,503, DE-A-2,944,601, JP-A-78/34044, 74/53435, 74/53436, 75/53372 und 75/122935.

Ueber ein zweiwertiges Q' können 2 Pyrazolonringe verknüpft werden und man erhält dann sogenannte Bis-Kuppler. Solche sind z.B. beschrieben im US-A-2,632,702, US-A-2,618,864, GB-A-968,461, GB-A-786,859, JP-A-76/37646, 59/4086, 69/16110, 69/26589, 74/37854 und 74/29638. Bevorzugt ist Y eine O-Alkoxyarylthio-Gruppe.

Wie vorstehend erwähnt können als Magentakuppler auch mit 5-gliedrigen Heterocyclen kondensierte Pyrazole - sogenannte Pyrazoloazole - verwendet werden. Deren Vorteile gegenüber einfachen Pyrazolen

ist, dass sie Farben von grösserer Formalin-Beständigkeit und reineren Absorptionsspektren aufweisen.

Man kann sie durch die allgemeine Formel XIV darstellen,

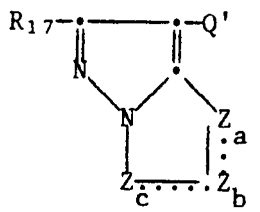

XIV

worin $Z_a$, $Z_b$ und $Z_c$ die Ergänzungen zu einem 5-gliedrigen Ring bedeuten, der bis zu 4 Stickstoffatome enthalten kann. Die Verbindungen können demgemäss Pyrazolo-imidazole, Pyrazolo-pyrazole, Pyrazolo-triazole oder Pyrazolo-tetrazole sein. $R_{17}$ und Q' haben dieselben Bedeutungen wie in Formel XIII.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in:

JP-A-86/28,947, JP-A-85/140,241, JP-A-85/262,160, JP-A-85/213,937, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Cyankuppler können z.B. Derivate von Phenol, von 1-Naphthol oder von Pyrazolochinazolon sein. Bevorzugt sind Strukturen der Formel XV,

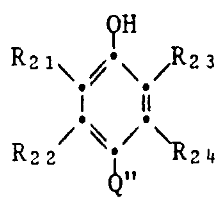

XV ,

worin $R_{21}$, $R_{22}$, $R_{23}$ und $R_{24}$ Wasserstoff, Halogen,Alkyl, Carbamoyl, Amido, Suflonamido, Phosphoramido oder Ureido sind. $R^{21}$ ist vorzugsweise H oder Cl, $R_{22}$ ist vorzugsweise eine Alkyl- oder Amidogruppe. $R_{23}$ ist vorzugsweise eine Amidogruppe und $R_{24}$ ist vorzugsweise Wasserstoff. Q" ist Wasserstoff oder eine Abgangsgruppe, die bei der Reaktion mit dem oxidierten Entwickler abgespalten wird. Eine ausführliche Aufzählung von Cyankupplern ist in der US-A-4,456,681 zu finden.

Beispiele von gebräuchlichen Cyankupplern sind die folgenden:

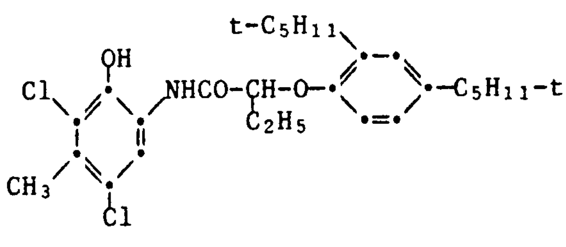

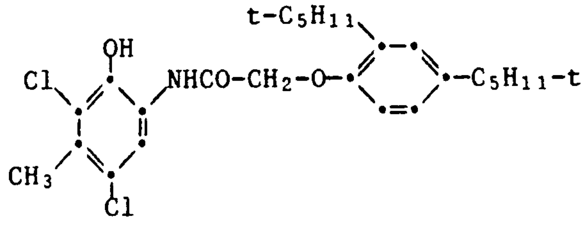

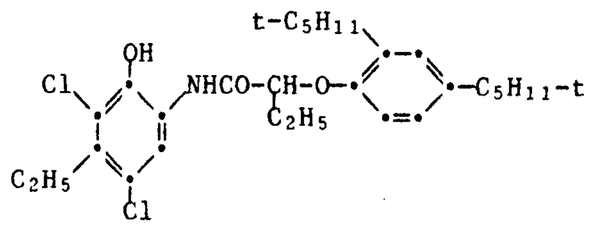

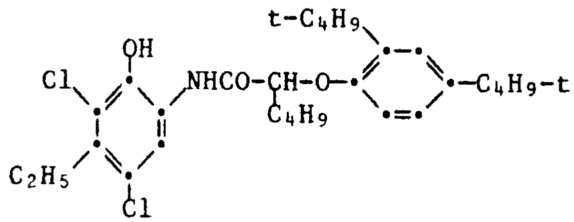

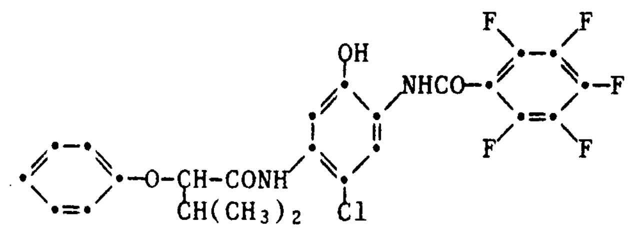

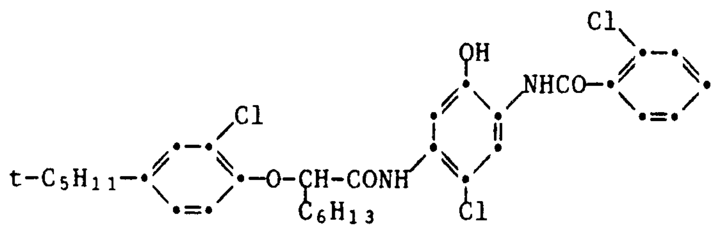

Weitere Beispiele von Cyankupplern sind in folgenden US-Patentschriften zu finden: 2,369,929, 2,423,730, 2,434,272, 2,474,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681.

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-methanesulphonamidoethylanilin, 3-Methyl-4-amino-N-ethyl-N-α-methoxyethyl-anilin, 3-α-Methansulphonamidoethyl-4-amino-N,N-diethylanilin, 3-Methoxy-4-amino-N-ethyl-N-α-hydroxyethylanilin, 3-Methoxy-4-amino-N-ethyl-N-α-methoxyethylanilin, 3-Acetamido-4-amino-N,N-diethylanilin, 4-Amino-N-N-dimethylanilin, N-Ethyl-N-α-[α-(α-methoxyethoxy)ethoxy]ethyl-3-methyl-4-aminoanilin, N-Ethyl-N-α-(α-methoxyethoxy)ethyl-3-methyl-4-aminoanilin sowie die Salze solcher Verbindungen, wie z.B. Sulfate, Hydrochloride oder Toluolsulfonate.

Die erfindungsgemässen Stabilisatoren können zusammen mit dem Farbkuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160 °C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutyl-keton, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Weitere Details über verwendbare hochsiedende Lösungsmittel sind in den folgenden Patentschriften zu finden.

Phosphate: GB-A-791,219, BE-A-755,248, JP-A-76/76739, 78/27449, 78/218,252, 78/97573, 79/148,113, 82/216,177, 82/93323 und 83/216,177.

Phthalate: GB-A-791,219, JP-A-77/98050, 82/93322, 82/216,176, 82/218,251, 83/24321, 83/45699, 84/79888.

Amide: GB-A-791,219, JP-A-76/105,043, 77/13600, 77/61089, 84/189,556, US-A-928,741.

Phenole: GB-A-820,329, FR-A-1,200,657, JP A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089 und BE-A-826,039.

Sonstige Verbindungen: JP-A-72/115,369, 72/130,258, 73/127,521, 73/76592, 77/13193, 77/36294, 79/95233 und Research Disclosure 82/21918.

Die Menge von hochsiedendem Lösungsmittel liegt im Bereich von 0,1 bis 300 %, vorzugsweise 10 bis 100 %, bezogen auf den Farbkuppler.

Die photographischen Schichten können ferner Farbschleier-Inhibitoren enthalten. Diese verhindern das Entstehen von Farbschleiern, wie sie beispielsweise durch Reaktion des Kupplers mit unabsichtlich oxidiertem Entwickler oder mit Nebenprodukten des Farbbildungsprozesses entstehen. Solche Farbschleierinhibitoren sind meist Hydrochinonderivate, können aber auch Derivate von Aminophenolen, von Gallussäure oder von Ascorbinsäure sein. Typische Beispiele hierfür sind in folgenden Patentschriften zu finden: US-A-2,360,290, 2,336,327, 2,403,721, 2,418,613, 2,675,314, 2,701,197, 2,704,713, 2,728,659, 2,732,300, 2,735,365; EP-A-124,877; JP-A-75/92988, 75/92989, 75/93928, 75/110,337, und 77/146,235.

Die photographischen Schichten können auch sogenannte DIR-Kuppler enthalten, die mit dem oxidierten Entwickler farblose Verbindungen ergeben. Sie werden zugesetzt zur Verbesserung der Schärfe und Körnigkeit der Farbbilder.

Die photographischen Schichten können auch UV-Absorber enthalten. Diese filtern das UV-Licht aus und schützen damit die Farbstoffe, die Kuppler oder sonstige Komponenten gegen Lichtabbau. Beispiele für solche UV-Absorber sind 2-(2-Hydroxyphenyl)-benztriazole, 2-Hydroxybenzophenone, Salicylsäureester, Acrylnitrilderivate oder Thiazoline. Solche UV-Absorber sind z.B. in folgenden Patentschriften näher aufgeführt: US-A-3,314,794, 3,352,681, 3,705,805, 3,707,375, 4,045,229, 3,700,455, 3,533,794, 3,698,907, 3,705,805, 3,738,837 und JP-A-71/2784. Bevorzugte UV-Absorber sind die 2-(2-Hydroxyphenol)-benztriazole.

Die photographischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind in den folgenden Patentschriften näher beschrieben: US-A-3,700,455, 3,591,381, 3,573,052, 4,030,931, 4,174,220, 4,178,184, 4,228,235, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246, 84/192,247, 84/204,039, 84/204,040, 84/212,837, 84/220,733, 84/222,836, 84/228,249, 86/2540, 86,8843, 86/18835, 86/18836, 87/11456, 87/42245, 87/62157, 86/6652 sowie in Research Disclosure 79/17804.

Die photographischen Schichten können auch gewisse Phosphor-III-Verbindungen, insbesondere Phosphite und Phosphonite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind in den folgenden Patentschriften näher beschrieben: US-A-4,407,935, US-A-4,436,811, EP-A-181,289, JP-A-73/32728, JP-A-76/1420 und JP-A-55/67741.

Die photographischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind in folgenden Patentschriften näher beschrieben: US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506; DE-A-2,853,865.

Die photographischen Schichten können auch Hydrochinonverbindungen enthalten. Diese wirken als Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind in folgenden Patentschriften näher beschrieben:

US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131, 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die photographischen Schichten können auch Derivate von Hydrochinonethern enthalten. Diese Verbindungen wirken als Lichtschutzmittel und sind besonders geeignet zur Stabilisierung von Magenta-Farbstoffen. Solche Verbindungen und deren Kombination mit anderen Additiven sind in folgenden Patentschriften näher beschrieben:

US-A 3,285,937, 3,432,300, 3,519,429, 3,476,772, 3,591,381, 3,573,052, 3,574,627, 3,573,050, 3,698,909, 3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,314,011, 4,273,864, 4,264,720, 4,279,990, 4,332,886, 4,436,165, 4,360,589, 4,416,978, 4,385,111, 4,459,015, 4,559,297; GB-A 1,347,556, 1,366,441, 1,547,392, 1,557,237, 2,135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,036, 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855 sowie in Research Disclosure 78/17051.

Das Bestreben farbphotographische Materialien in noch kürzerer Zeit zu entwickeln und dabei Chemikalien zu verwenden, die einfacher in der Handhabung und weniger umweltbelastend sind, hat zu erheblichen Beschränkungen in der Wahl der Komponenten des Systems geführt. So werden als Silberhalogenid-Emulsionen solche verwendet, die weitgehend oder ganz auf Silberchlorid basieren, wodurch die Entwicklungszeit verkürzt wird. Weiterhin wurde gefunden, dass Entwicklersysteme weitgehend oder ganz ohne Benzylalkohol verwendet werden können, ohne dass die Farbdichte verringert wird. Dies ermöglicht Entwicklerkonzentrate aus weniger Bestandteilen, mit kürzeren Mischungszeiten und geringerer Toxizität des verbrauchten Entwicklers. Um dieses Ziel der Verkürzung der Entwicklungszeit und der Reduktion des Benzylalkohols zu erreichen, können folgende Zusätze verwendet werden:

a) N-substituierte Hydroxylamine als Antioxidantien anstelle der üblichen Hydroxylamine,

b) Entwicklungsbeschleuniger, wie z.B. 1-Aryl-3-pyrazolone, Hydrazinderivate, quartäre Ammonium- und Phosphoniumverbindungen oder Polyoxyalkylenverbindungen,

c) Triethanolamin als Teerbekämpfer,

d) Lithiumsalze, z.B. solche von Polystyrolsulfonaten,

e) aromatische Polyhydroxylverbindungen, wie z.B. 5,6-Dihydroxy-1,2,4-benzoltrisulfonsäure-Natriumsalz.

Die Verbindungen der Formel I und II sind auch in solchen schnellentwickelbaren Systemen brauchbar, wie in photographischen Schichten auf Basis von Silberchlorid-Emulsionen, und in Systemen, die ganz oder weitgehend ohne Benzylalkohol entwickelt werden.

Die folgenden Beispiele zeigen die Herstellung und Verwendung der neuen Verbindungen im Detail. Darin bedeuten Teile und Prozente Gewichtsteile und Gewichtsprozente. Die Temperaturen sind in °C angegeben.

Beispiel 1: In 350 ml Toluol werden 27,0 g cis-2,6-Diphenylthian-4-ol (Smp. 155-156°, hergestellt nach C.A.R. Baxter, D.A. Whiting/J. Chem. Soc. 1968, 1176) und 27,8 g 3-(3,5-Di-tert.butyl-4-hydroxyphenyl)-propionsäure unter Rühren eingetragen. Nach Zugabe von 1,5 g p-Toluolsulfonsäure wird das Reaktionsgemisch 12 Stunden am Wasserabscheider zum Rückfluss erhitzt. Nach dem Abkühlen wird die Reaktionslösung dreimal mit je 100 ml Wasser gewaschen, über $Na_2SO_4$ getrocknet und im Vakuum eingedampft. Der Rückstand wird aus Ethanol umkristallisiert. Man erhält das 4-[3-(3,5-Di-tert.butyl-4-hydroxyphenyl)-propionyloxy]-cis-2,6-diphenylthian, das bei 110° schmilzt (Verbindung Nr. 10).

In analoger Weise werden aus den jeweiligen Thian-alkoholen und phenolischen Carbonsäuren die in Tabelle 1 aufgeführten Ester hergestellt.

Hierin bedeutet: Ph = Phenyl
+ = tert.Butyl

Tabelle 1

| Strukturformel | Verbindung Nr. | Schmelzpunkt |
|---|---|---|
| 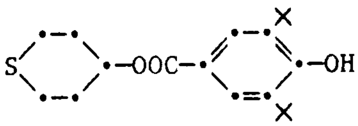 | 1 | 114° |
| 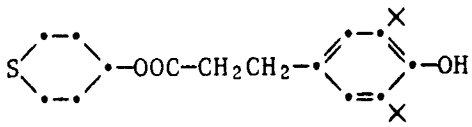 | 2 | 76° |
| 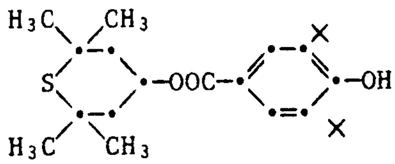 | 3 | 149° |
| 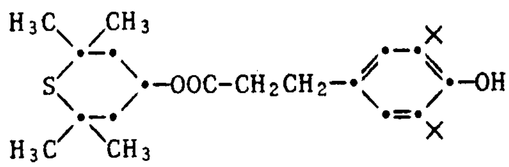 | 4 | 82° |
| 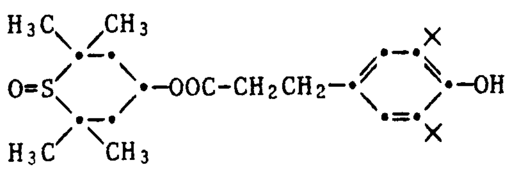 | 5 | 114° |

| | | | |
|---|---|---|---|
| 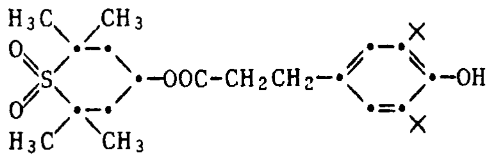 | | 6 | 158° |
| 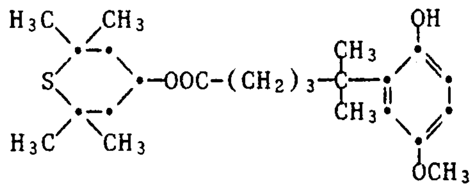 | | 7 | Oel |
| 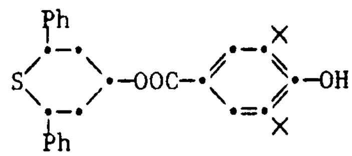 | cis<br>trans | 9a<br>9b | 102°<br>184° |
| 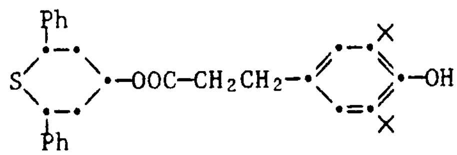 | | 10 | 110° |
| 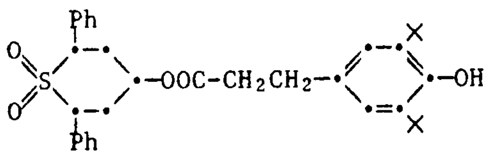 | | 12 | 188-190° |
| 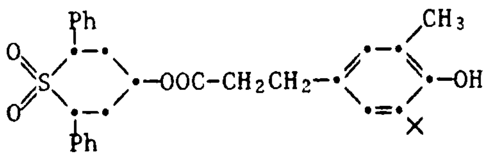 | | 13 | 116-118° |
| 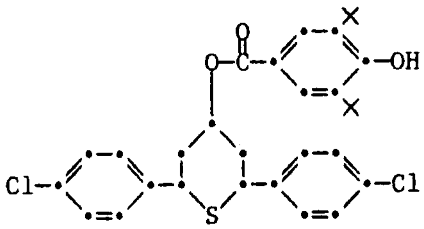 | | 34 | Harz |
| 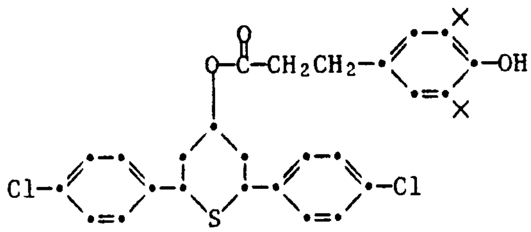 | | 35 | 157° |

| | | |
|---|---|---|
| 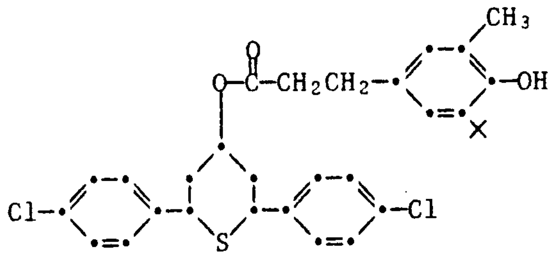 | 36 | Harz |
| 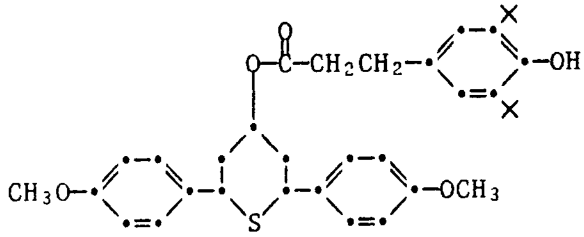 | 37 | Harz |
| 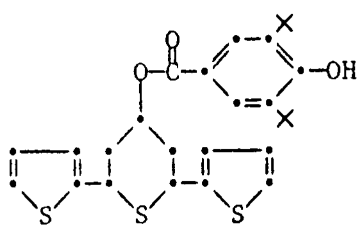 | 42 | 144-146° |
| 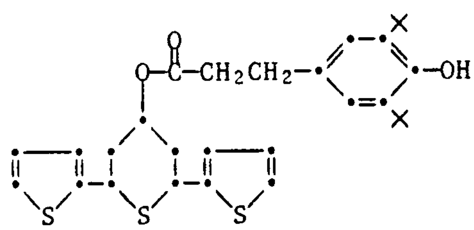 | 43 | 97- 99° |

<u>Beispiel 2</u>: <u>Umesterung</u>

115,4 g 3-Ethyl-3-hydroxymethyl-8,10-diphenyl-1,5-dioxa-9-thiaspiro[5.5]undecan und 88,6 g 3-(3,5-Di-tert.butyl-4-hydroxyphenyl)-propionsäuremethylesterwerden in 800 ml Benzin (Siedebereich 110-140°) eingetragen. Man destilliert ca. 50 ml Benzin ab und lässt die Lösung auf ca. 80° abkühlen. Nach Zusatz von 0,3 g Lithiumamid wird das Reaktionsgemisch unter einem schwachen Stickstoffstrom erwärmt bis das gebildete Methanol - zusammen mit etwas Benzin - überdestilliert. Nach ca. 4 Stunden lässt man etwas abkühlen und gibt 0,8 g Eisessig zu. Nach 10 Minuten gibt man 10 g Tonsil zu, rührt weitere 5 Minuten und filtriert. Aus dem Filtrat kristallisiert beim Abkühlen das 3-[3-(3,5-Di-tert.butyl-4-hydroxyphenyl)-propionyloxymethyl]-3-ethyl -8,10-diphenyl-1,5-dioxa-9-thiaspiro[5.5]undecan, welches nach dem Trocknen bei 122° schmilzt (Verbindung Nr. 19).

In analoger Weise werden aus den jeweiligen Thianalkoholen und Phenolcarbonsäuremethylestern die in Tabelle 2 aufgeführten Ester hergestellt.

Hierin bedeutet: Ph = Phenyl
+ = tert.Butyl

Tabelle 2

| Strukturformel | Verbindung Nr. | Schmelzpunkt |
|---|---|---|
| 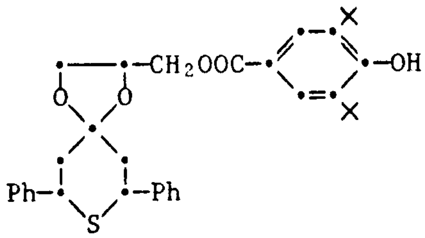 | 15 | Harz |
| 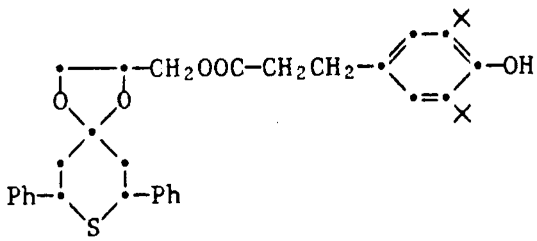 | 16 | Harz |
| 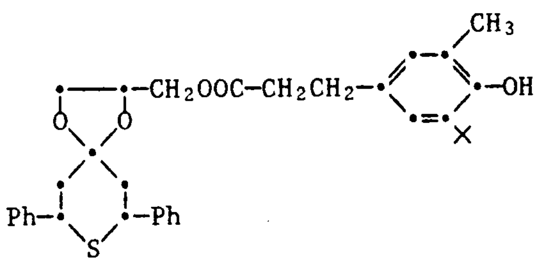 | 17 | Harz |
| 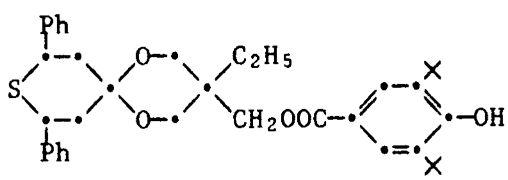 | 18 | 142-144° |

| | | |
|---|---|---|
| 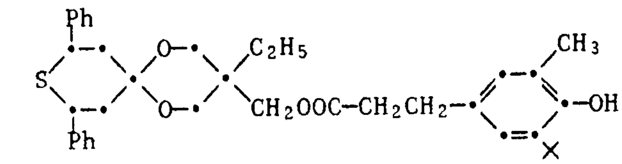 | 20 | 123° |
| 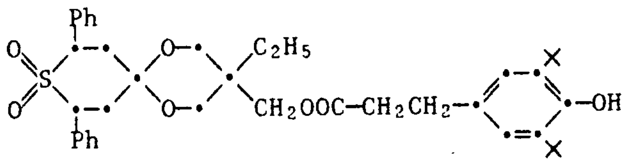 | 21 | Harz |
| 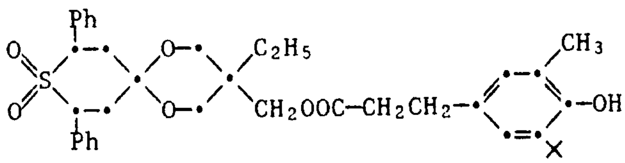 | 22 | Harz |
| 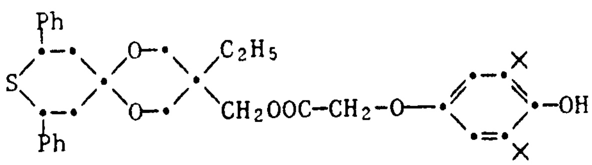 | 23 | Oel |
| 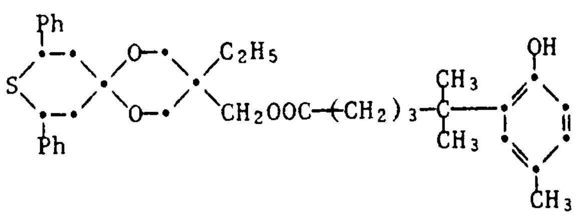 | 26 | Oel |
| 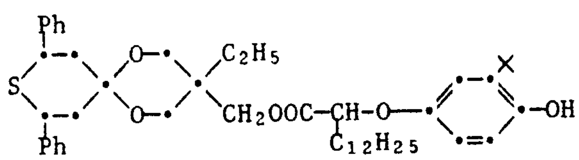 | 27 | Oel |
| 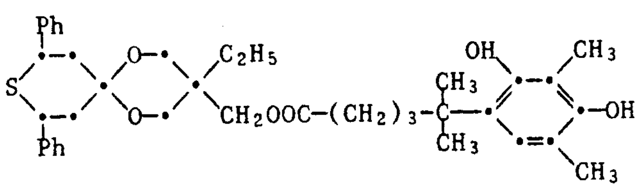 | 28 | Oel |
| 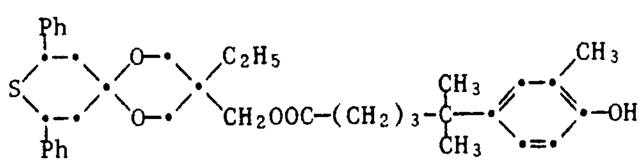 | 29 | Oel |

| | | |
|---|---|---|
| 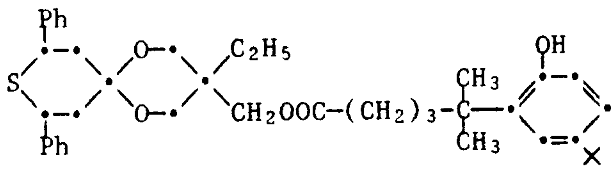 | 30 | Oel |
| 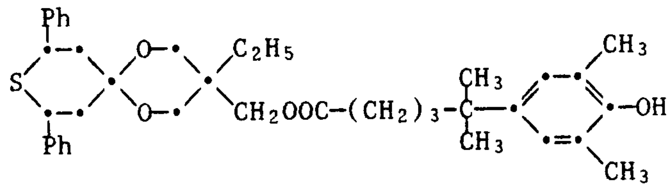 | 31 | Oel |
| 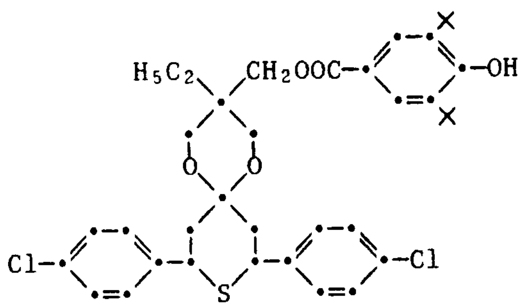 | 34 | 191-193° |
| 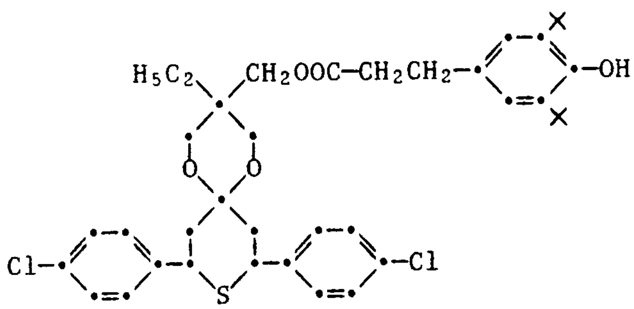 | 38 | Harz |
| 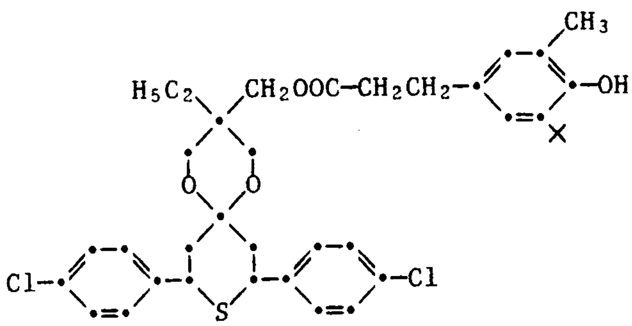 | 40 | Harz |

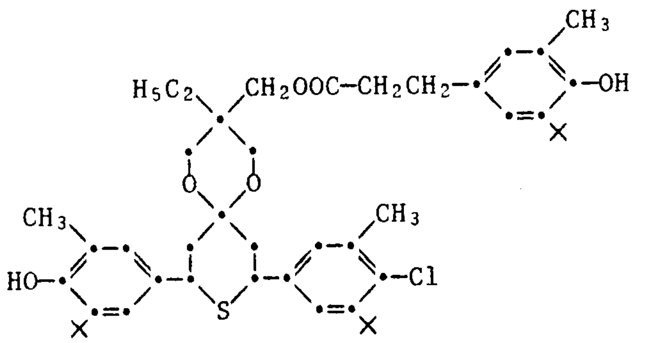

41 Harz (85-95°)

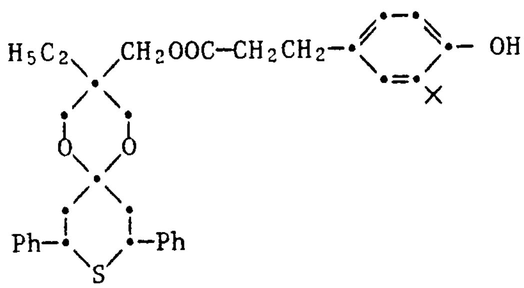

45 Harz

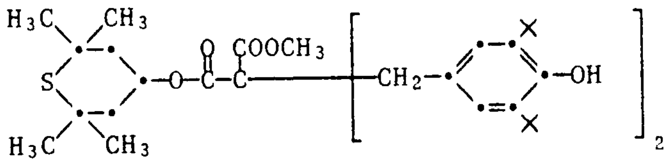

50 163°

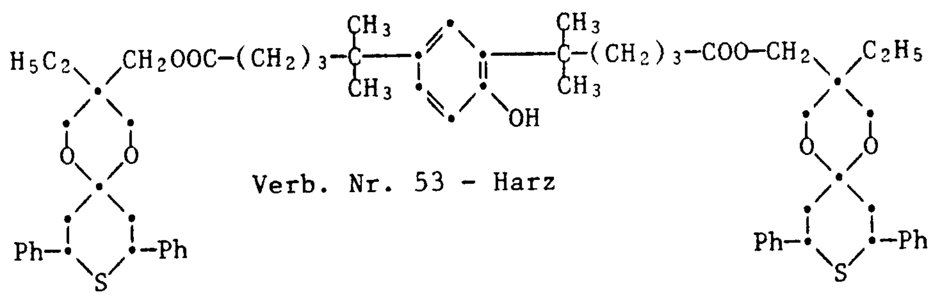

Verb. Nr. 53 - Harz

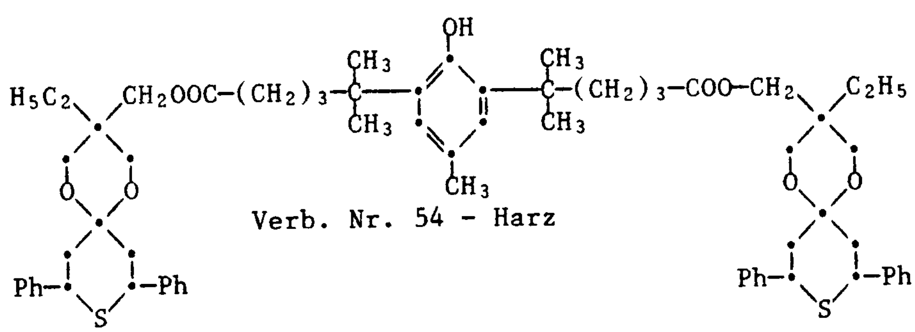

Verb. Nr. 54 - Harz

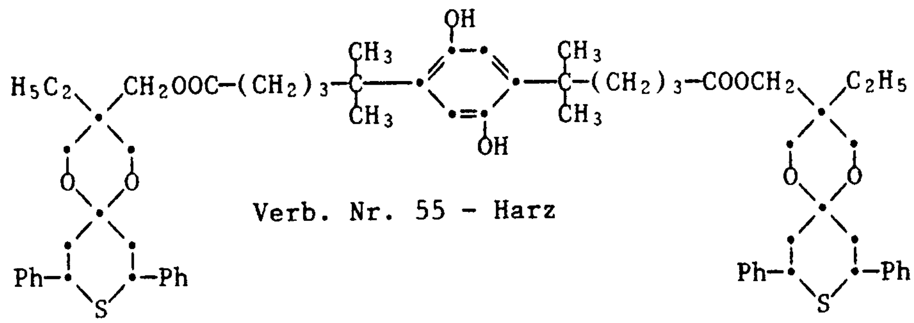

Verb. Nr. 55 - Harz

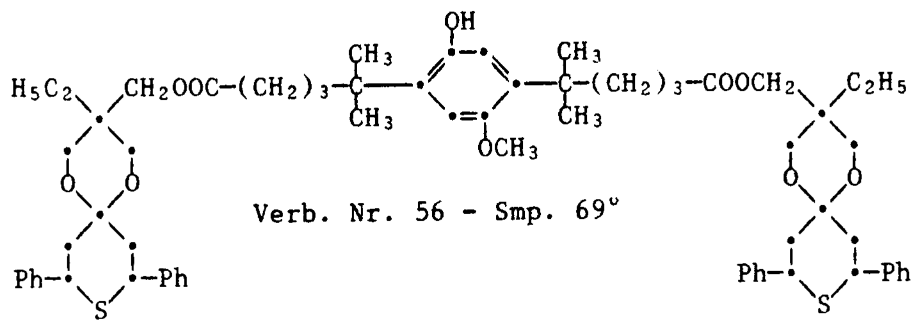

Verb. Nr. 56 - Smp. 69°

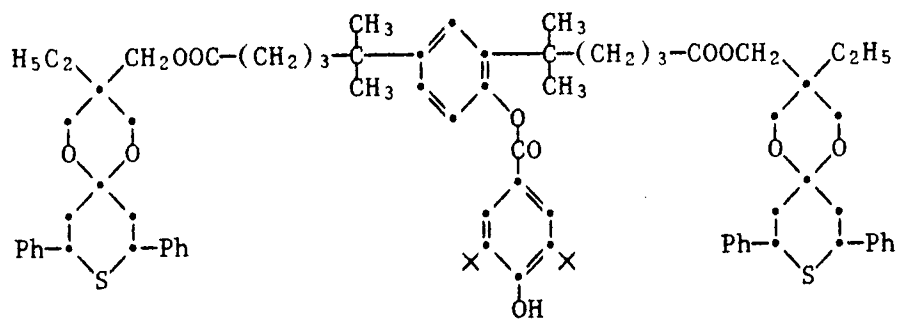

Verb. Nr. 57 - viskoses Oel

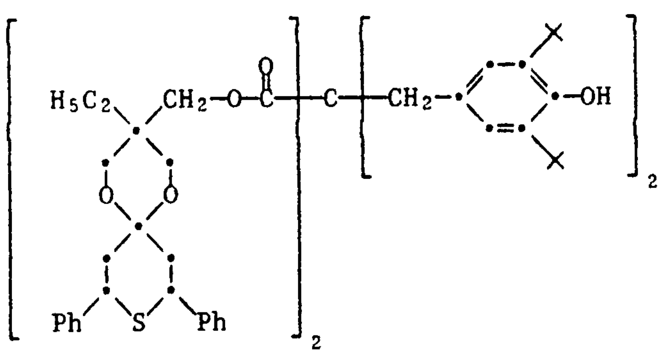

Verb. Nr. 59 - Smp. 168°

Beispiel 3: Acylhydrazone

12,5 g 3-(3-Methyl-4-tert.butyl-4-hydroxyphenyl)-propionsäurehydrazid und 13,6 g 2,6-Diphenyl-4-oxothian werden in 300 ml Ethanol 3 Std. zum Rückfluss erwärmt. Das beim Abkühlen auskristallisierende Rohprodukt wird abfiltriert und aus 500 ml Xylol umkristallisiert. Das erhaltene N-[$\beta$-(3-Methyl-5-tert.butyl-4-hydroxyphenyl)-propionylamido]-2,6-diphenyl-4-iminothian der Formel

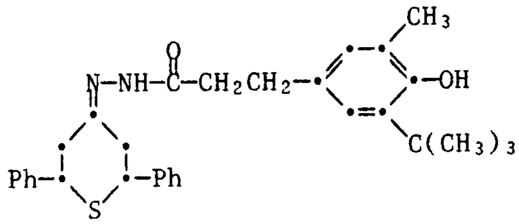

schmilzt nach Trocknung im Vakuum bei 211 -212° (Verbindung Nr. 48).

In analoger Weise wird aus 14,6 g 3-(3,5-Di-tert.butyl-4-hydroxyphenyl)-propionsäurehydrazid und 13,4 g 2,6-Diphenyl-4-oxothian das N-[β-(3,5-Di-tert.butyl-4-hydroxyphenyl)-propionylamido]-2,6-diphenyl-4-iminothian hergestellt, das bei 266° schmilzt (Verbindung Nr. 49).

Beispiel 4: Hydroxybenzylierung von Malonaten

19 g Di-(2,2,6,6-tetramethylthian-4-yl)-malonat und 33,5 g N,N-Diethyl-S-(3,5-di-tert.butyl-4-hydroxybenzyl)-dithiocarbaminat werden in 150 ml Isopropanol auf 50° erwärmt. Bei dieser Temperatur wird eine Lösung von 3,6 g NaOH in 20 ml Wasser innerhalb 30 Minuten zugetropft. Dabei beginnt das Produkt zu kristallisieren. Das Gemisch wird 1 h auf 50° und 3 h zum Rückfluss erwärmt und dann auf Raumtemperatur abgekühlt. Das ausgefallene Produkt wird abfiltriert und aus Aceton/Acetonitril umkristallisiert. Der erhaltene Bis-(3,5-di-tert.butyl-4-hydroxybenzyl)-malonsäure-di(2,2,6,6-tetramethylthian-4-yl)ester der Formel

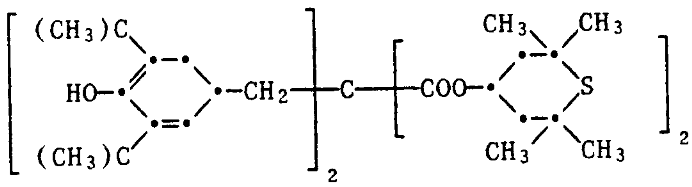

schmilzt bei 220° (Verbindung Nr. 51).

Beispiel 5: Acylierung der Phenolgruppe

1,5 g 3-[3-(3-tert.Butyl-5-methyl-4-hydroxyphenyl)-propionyloxymethyl]-3-methyl-8,10-diphenyl-1,5-dioxa-9-thiaspiro[5.5]undecan werden in 20 ml Toluol gelöst. Dazu gibt man 2 ml Dimethylformamid und 0,63 g Trimethylamin. Unter Kühlung auf 0° werden 0,6 g Oxalsäuremonomethylesterchlorid in 10 ml Toluol getropft. Das Gemisch wird 6 h bei Raumtemperatur gerührt. Dann werden 20 ml Wasser zugegeben, die organische Phase abgetrennt, getrocknet und eingedampft. Als Rückstand verbleibt das rohe Produkt der Formel

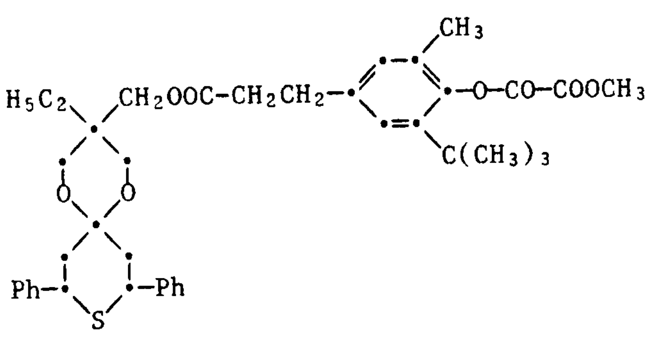

als farbloses Oel (Verbindung Nr. 46).

In analoger Weise erhält man bei Umsetzung von 1,43 g 3-[5-(3,5-Dimethyl-3-hydroxyphenyl)-5-methylhexanoyloxymethyl]-3-ethyl-8,10-diphenyl-1,5-dioxa-9-thiaspiro[5.5]undecan die Verbindung der Formel

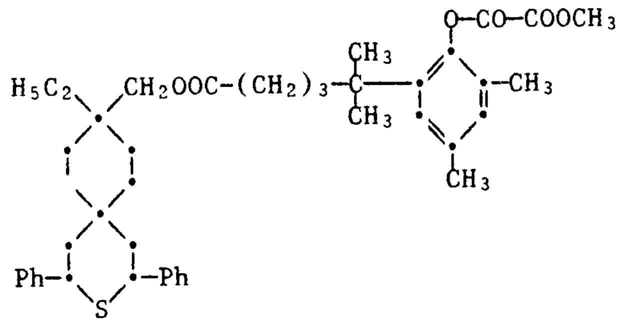

als gelbliches Oel (Verbindung Nr. 52).

Beispiel 6: Silylierung der Phenolgruppe

1,5 g 3-[3-(3-tert.Butyl-5-methyl-4-hydroxyphenyl)-propionyloxymethyl]-8,10 -diphenyl-1,5-dioxa-9-thiaspiro[5.5]-undecan und 0,94 g 1,8-Diazabicyclo[5.4.0]undec-7-en werden in 20 ml Toluol gelöst. Unter Kühlung auf 0° werden 0,54 g Trimethylchlorsilan in 10 ml Toluol zugetropft. Das Gemisch wird 6 h bei Raumtemperatur gerührt, dann werden 20 ml Wasser zugesetzt und die organische Phase abgetrennt, getrocknet und eingedampft. Der ölige Rückstand wird durch Chromatographie an einer $Al_2O_3$-Säule gereinigt. Als Elutionsmittel wird dabei Hexan/Ethylacetat 9:1 verwendet. Man erhält die Verbindung der Formel

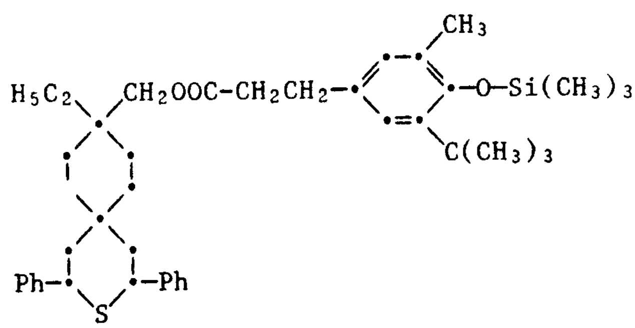

als farbloses Oel (Verbindung Nr. 47).

Beispiel 7: Stabilisierung einer Gelbschicht

0,087 g des Gelb-Kupplers der Formel

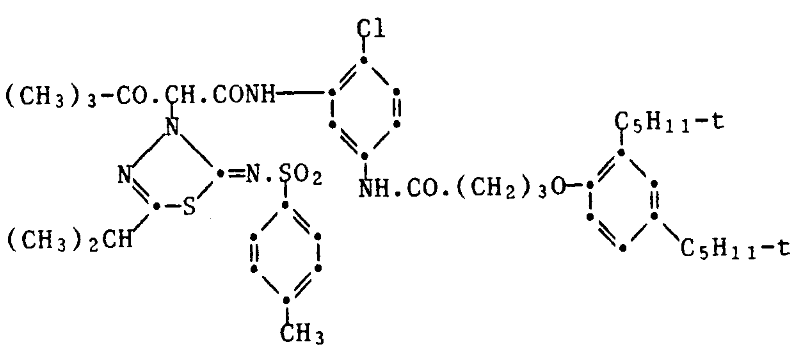

und 0,029 g eines der in den nachfolgenden Tabellen angegebenen Lichtschutzmittel werden in 2 ml eines Gemisches von Dibutylphthalat/Aethylacetat (1,5 g/100 ml) gelöst. Zu 1 ml dieser Lösung gibt man 9 ml einer 2,3%igen wässrigen Gelatinelösung, die auf einen pH-Wert von 6,5 eingestellt ist und 1,744 g/l

Netzmittel ®Nekal BX (Diisobutylnaphthalin-sulfonsäure-Na-salz) enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 6 g pro Liter und 1 ml einer 0,7%igen wässrigen Lösung von Cyanursäuredichlord als Gelatine-Härter und vergiesst auf ein ein 13x18 cm kunststoffbeschichtetes Papier. Nach einer Härtungszeit von 7 Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Atlas Weather-Ometer hinter einem UV-Filter (Kodak 2C) mit einer 2500 W Xenonlampe mit total 60 Kilojoule per $cm^2$ bestrahlt.

In den folgenden Tabellen 3 und 4 sind die prozentualen Farbdichteabnahmen bei einer ursprünglichen Farbdichte von 1,0 angegeben.

Tabelle 3

| Stabilisator Verbindung Nr. | Dichteverlust in % (hinter UV-Filter) |
|---|---|
| keiner | 23 |
| 18 | 11 |
| 42 | 12 |
| 43 | 11 |
| 44 | 12 |

Tabelle 4

| Stabilisator Verbindung Nr. | Dichteverlust in % (hinter UV-Filter) |
|---|---|
| keiner | 27 |
| 9a | 10 |
| 10 | 9 |
| 12 | 15 |
| 13 | 11 |
| 15 | 8 |
| 16 | 11 |
| 17 | 8 |
| 19 | 9 |
| 20 | 8 |
| 21 | 15 |
| 22 | 12 |
| 26 | 12 |
| 28 | 7 |
| 29 | 13 |
| 30 | 12 |
| 31 | 12 |
| 34 | 12 |
| 35 | 9 |
| 36 | 10 |
| 37 | 14 |
| 38 | 8 |
| 40 | 8 |
| 41 | 8 |
| 46 | 11 |
| 47 | 13 |
| 48 | 14 |
| 53 | 12 |
| 54 | 14 |
| 55 | 13 |
| 56 | 10 |
| 57 | 14 |
| 58 | 11 |
| 59 | 9 |

Beispiel 8: 0,091 g des Gelb-Kupplers der Formel

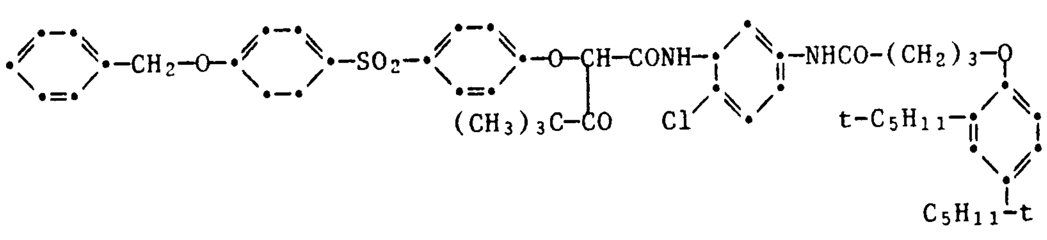

und 0,027 g eines in der folgenden Tabelle angegebenen Lichtschutzmittels werden in 2 ml eines Gemisches von Dibutylphthalat/Aethylacetat (1,5 g/100 ml) gelöst. Zu 1 ml dieser Lösung gibt man 9 ml einer 2,3%igen wässrigen Gelatinelösung, die auf einen pH-Wert von 6,5 eingestellt ist und 1,744 g/l Netzmittel ®Nekal BX enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 6 g pro Liter und 1 ml einer 0,7%igen wässrigen Lösung von Cyanursäuredichlorid als Gelatine-Härter und vergiesst auf 13x18 cm kunststoffbeschichtetes Papier. Nach einer Härtungszeit von 7

Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Atlas Weather-Ometer hinter einem UV-Filter (Kodak 2C) mit einer 2500 Xenonlampe mit total 60 Kilojoule per $cm^2$ bestrahlt.

In Tabelle 5 sind die prozentualen Farbdichteabnahmen bei einer ursprünglichen Farbdichte von 1,0 angegeben.

Tabelle 5

| Stabilisator Verbindung Nr. | Dichteverlust in % (hinter UV-Filter) |
|---|---|
| keiner | 18 |
| 9a | 10 |
| 10 | 10 |
| 19 | 8 |
| 22 | 10 |
| 34 | 9 |
| 35 | 9 |
| 38 | 11 |
| 40 | 8 |

Beispiel 9: 0,076 g des Gelb-Kupplers der Formel

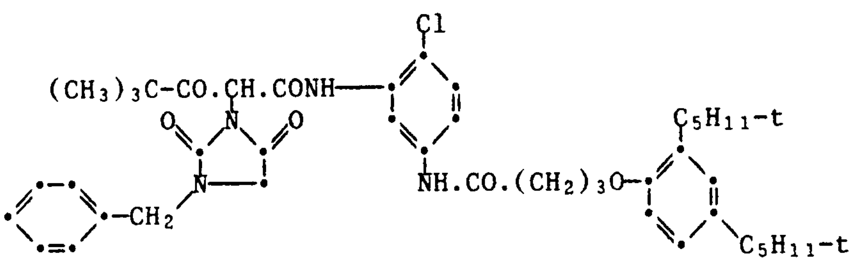

und 0,023 g eines in der nachfolgenden Tabelle angegebenen Lichtschutzmittels werden in 2 ml eines Gemisches von Dibutylphthalat/Aethylacetat (1,5 g/100 ml) gelöst. Zu 1 ml dieser Lösung gibt man 9 ml einer 2,3%igen wässrigen Gelatinelösung, die auf einen pH-Wert von 6,5 eingestellt ist und 1,744 g/l ®Nekal BX enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 6 g pro Liter und 1 ml einer 0,7%igen wässrigen Lösung von Cyanursäuredichlorid als Gelatine-Härter und vergiesst auf ein 13x18 cm kunststoffbeschichtetes Papier. Nach einer Härtungszeit von 7 Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Atlas Weather-Ometer hinter einem UV-Filter (Kodak 2C) mit einer 2500 W Xenonlampe mit total 60 Kilojoule per $cm^2$ bestrahlt.

In der folgenden Tabelle 6 sind die prozentualen Farbdichteabnahmen bei einer ursprünglichen Farbdichte von 1,0 angegeben.

Tabelle 6

| Stabilisator Verbindung Nr. | Dichteverlust in % (hinter UV-Filter) |
|---|---|
| keiner | 21 |
| 19 | 8 |
| 34 | 10 |
| 36 | 10 |
| 38 | 9 |
| 40 | 7 |

Beispiel 10: 0,038 g des Magenta-Kupplers der Formel

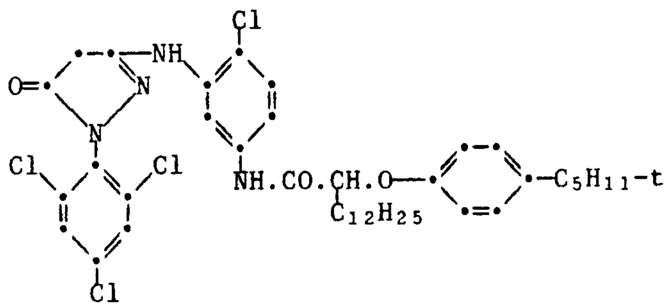

und 0,013 g eines in Tabelle 7 angegebenen Lichtschutzmittels werden in 2 ml eines Gemisches von Trikresylphosphat/Aethylacetat (1 g/100 ml) gelöst. Zu 1 ml dieser Lösung gibt man 9 ml einer 2,3%igen wässrigen Gelatinelösung, die auf einen pH-Wert von 6,5 eingestellt ist und 0,436 g/l ®Nekal BX enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 6 g pro Liter und 1 ml einer 0,7%igen wässrigen Lösung von Cyanursäuredichlorid als Gelatine-Härter und vergiesst auf ein 13x18 cm kunststoffbeschichtetes Papier. Nach einer Härtungszeit von 7 Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Klimaschrank 28 Tage bei 75°C und 60 % rel. Luftfeuchtigkeit gelagert. Die folgende Tabelle 7 zeigt die prozentuelle Farbdichtezunahme bei einer ursprünglichen Farbdichte von 1.0.

Tabelle 7

| Stabilisator Verbindung Nr. | Dichtezunahme in % (hinter UV-Filter) |
|---|---|
| keiner | 41 |
| 9a | 28 |
| 18 | 28 |
| 42 | 31 |

Beispiel 11: 0,025 g des Cyan-Kupplers der Formel

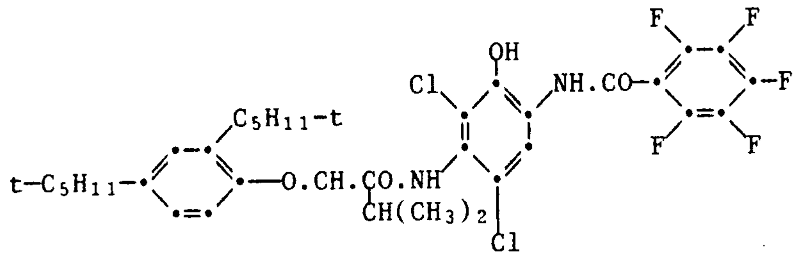

und 0,025 g eines der in der Tabelle 8 angegebenen Lichtschutzmittel werden in 2 ml eines Gemisches von Dibutylphthalat/Aethylacetat (0,8 g/100 ml) gelöst. Zu 1 ml dieser Lösung gibt man 9 ml einer 2,3%igen wässrigen Gelatinelösung, die auf einen pH-Wert von 6,5 eingestellt ist und 0,872 g/l ®Nekal BX enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 3 g pro Liter, 1 ml einer 0,7%igen wässrigen Lösung von Cyanursäuredichlorid als Gelatine-Härter und vergiesst auf ein 13x18 cm kunststoffbeschichtetes Papier. Nach einer Härtungszeit von 7 Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Atlas Weather-Ometer hinter einem UV-Filter (Kodak 2C) mit einer 2500 W Xenonlampe mit total 60 Kilojoule per $cm^2$ bestrahlt.

In Tabelle 8 sind die prozentualen Farbdichteabnahmen bei einer ursprünglichen Farbdichte von 1,0 angegeben.

Tabelle 8

| Stabilisator Verbindung Nr. | Dichteverlust in % (hinter UV-Filter) |
|---|---|
| keiner | 29 |
| 18 | 19 |
| 20 | 17 |

Beispiel 12: 0,025 g des Cyan-Kupplers der Formel

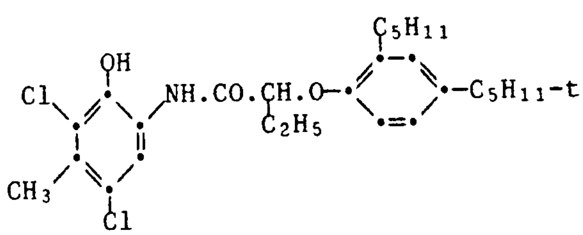

und 0,025 g eines der in der Tabelle 9 angegebenen Lichtschutzmittel werden in 2 ml eines Gemisches von Dibutylphthalat/Aethylacetat (0,8 g/100 ml) gelöst. Zu 1 ml dieser Lösung gibt man 9 ml einer 2,3%igen wässrigen Gelatinelösung, die auf einen pH-Wert von 6,5 eingestellt ist und 0,872 g/l ®Nekal BX enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 3 g pro Liter, 1 ml einer 0,7%igen wässrigen Lösung von Cyanursäuredichlorid als Gelatine-Härter und vergiesst auf ein 13x18 cm kunststoffbeschichtetes Papier. Nach einer Härtungszeit von 7 Tagen werden die Proben durch einen Silber-Stufenkeil mit 125 Lux•s belichtet und anschliessend im Ektaprint 2-Prozess der Firma Kodak verarbeitet.

Die so erhaltenen Farbstufenkeile werden in einem Klimaschrank 28 Tage bei 75°C und 60 % rel. Luftfeuchtigkeit gelagert.

In Tabelle 9 sind die prozentualen Farbdichteabnahmen bei einer ursprünglichen Farbdichte von 1,0 angegeben.

Tabelle 9

| Stabilisator Verbindung Nr. | Dichteverlust in % (hinter UV-Filter) |
|---|---|
| keiner | 50 |
| 18 | 30 |
| 9b | 31 |

Beispiel 13: 0.031 g des Magenta-Kupplers der Formel

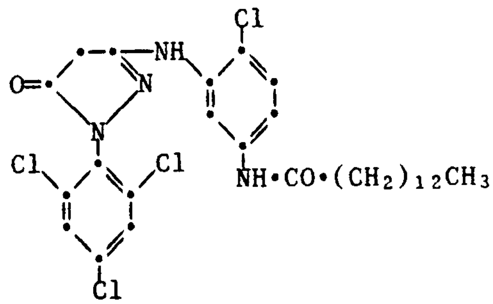

und die Menge eines der in der nachfolgenden Tabelle 10 angegebenen Lichtschutzmittel (resp. Lichtschutzmittelgemische) werden in 2 ml eines Gemisches von Trikresylphosphat/Aethylacetat (0,769 g/100 ml) gelöst. Zu 1.0 ml dieser Lösung gibt man 9.0 ml einer 2.3 %igen wässrigen Gelatinelösung, die auf einen pH-Wert von 6.5 eingestellt ist und 0.436 g/lt Netzmittel® Nekal BX (Diisobutylnaphthalin-sulfonsäure-Na-salz) enthält. Danach emulgiert man mit Ultraschall 3 Minuten.

Zu 5.0 ml der so erhaltenen Kuppleremulsion gibt man 2 ml einer Silberbromid-Emulsion mit einem Silbergehalt von 6 g pro Liter, 1.0 ml einer 0.7 %igen wässrigen Lösung von Cyanursäuredichlorid als Gelatinehärter und vergiesst es auf ein 13x18 cm kunststoffbeschichtetes Papier. Nach einer Härtungszeit von 7 Tagen werden die Proben durch einen Silber-Stufenkeil mit 125 Lux s belichtet und anschliessend im Ektaprint 2-Prozess der Firma Kodak verarbeitet.

Die so erhaltenen Farbstufenkeile werden in einem Atlas Weather-Ometer hinter einem UV-Filter (Kodak 2C) mit einer 3500 W Xenonlampe mit total 60 Kilojoule per $cm^2$ bestrahlt.

In der folgenden Tabelle 10 ist die Zunahme der Gelbfarbdichte im nicht belichteten Teil des Stufenkeils aufgeführt ($\Delta D_B$).

Tabelle 10:

| Stabilisator Verbindung Nr. | Menge (g) | Δ $D_B$ |
|---|---|---|
| Keines | - | 16 % |
| 10 | 0,011 | 7 % |
| 19 | 0,011 | 8 % |
| 10 + Costabilisator A | 0,0055<br>0,0055 | 6 % |
| 19 + Costabilisator A | 0,0055<br>0,0055 | 5 % |

Costabilisator A = phenolische Verbindung der Formel

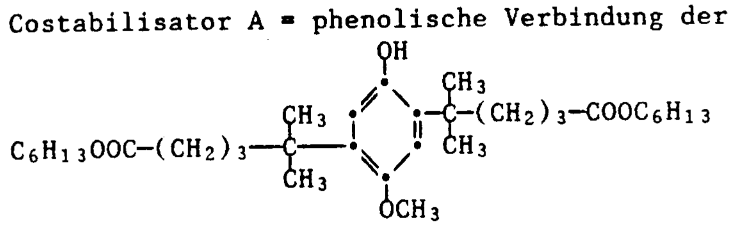

## Patentansprüche

1. Eine Verbindung der Formel I oder II,

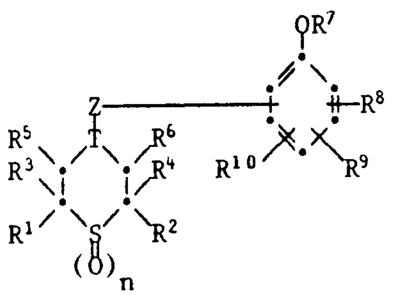

(I)

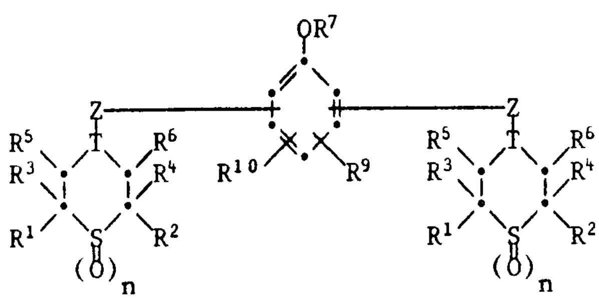

(II)

worin n 0, 1 oder 2 ist,
$R^1$ und $R^2$ unabhängig voneinander Wasserstoff oder Methyl bedeuten,
$R^3$ und $R^4$ unabhängig voneinander Wasserstoff, $C_1$-$C_4$-Alkyl, Phenyl, Thienyl oder durch 1 oder 2 $C_1$-$C_8$-Alkylgruppen, Cyclohexyl, Phenyl, $C_7$-$C_9$-Phenylalkyl, Hydroxyl, $C_1$-$C_{18}$-Alkoxy oder Halogen substituiertes Phenyl bedeuten,
$R^5$ und $R^6$ unabhängig voneinander Wasserstoff, $C_1$-$C_4$-Alkyl, Phenyl, -COO($C_1$-$C_{18}$-Alkyl), -CO-$CH_3$ oder -CO-Phenyl bedeuten,

$R^7$ Wasserstoff, $C_1$-$C_8$-Alkyl oder eine der Gruppen -CO-$R^{11}$, -CO-COO($C_1$-$C_4$-Alkyl), -$SO_2$-$R^{12}$, -CON-($R^{13}$)($R^{14}$), -Si($R^{15}$)($R^{16}$)($R^{17}$) oder

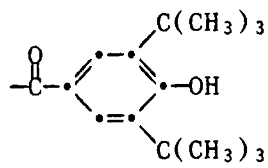

bedeutet,
$R^8$ und $R^9$ unabhängig voneinander Wasserstoff, $C_1$-$C_{12}$-Alkyl, $C_7$-$C_9$-Phenylalkyl, $C_5$-$C_8$-Cycloalkyl oder Phenyl bedeuten,
$R^{10}$ Wasserstoff, -$OR^7$ oder eine Gruppe der Formel III bedeutet,

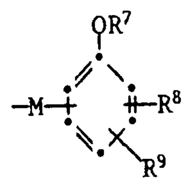

III

worin M eine direkte Bindung, -O-, -S-, -S-S-, -$CH_2$-, -CH($C_1$-$C_8$-Alkyl)- oder -C($CH_3$)$_2$- bedeutet,
$R^{11}$ $C_1$-$C_{20}$-Alkyl, $C_3$-$C_{20}$-Alkenyl, $C_5$-$C_{12}$-Cycloalkyl, $C_7$-$C_{13}$-Phenylalkyl oder $C_6$-$C_{10}$-Aryl bedeutet,
$R^{12}$ $C_1$-$C_{12}$-Alkyl, $C_6$-$C_{10}$-Aryl oder $C_7$-$C_{24}$-Alkylaryl bedeutet,
$R^{13}$ Wasserstoff, $C_1$-$C_{12}$-Alkyl oder Cyclohexyl bedeutet,
$R^{14}$ $C_1$-$C_{12}$-Alkyl, $C_6$-$C_{10}$-Aryl, durch $C_1$-$C_{12}$-Alkyl substituiertes $C_6$-$C_{10}$-Aryl oder Cyclohexyl bedeutet, oder $R^{13}$ und $R^{14}$ zusammen mit dem N-Atom einen 5- oder 6-gliedrigen gesättigten heterocyclischen Ring bilden,
$R^{15}$, $R^{16}$ und $R^{17}$ unabhängig voneinander $C_1$-$C_{12}$-Alkyl, $C_3$-$C_{12}$-Alkenyl, Phenyl, Cyclohexyl oder Benzyl bedeuten.
T eine dreiwertige Gruppe ist, die den Ring zu einem Thianring ergänzt und eine der folgenden Gruppen ist:
$>$CH-O-, $>$C=N-NH-,

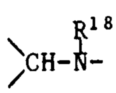 , 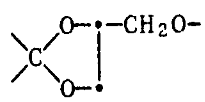 oder 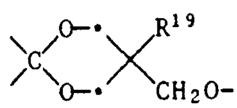 ,

worin $R^{18}$ Wasserstoff, $C_1$-$C_{12}$-Alkyl, Benzyl, Cyclohexyl oder Phenyl ist und $R^{19}$ Wasserstoff oder $C_1$-$C_4$-Alkyl ist,
Z ein zweiwertiges Bindeglied zwischen T und dem Phenolrest ist und eine der folgenden Gruppen ist:

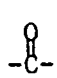 , 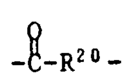 , 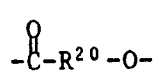 oder 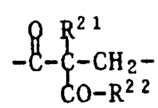 ,

deren Carbonylgruppe an T gebunden ist und worin
$R^{20}$ $C_1$-$C_{14}$-Alkylen bedeutet,
$R^{21}$ Wasserstoff, $C_1$-$C_{12}$-Alkyl, Phenyl, $C_7$-$C_9$-Phenylalkyl oder eine Gruppe der Formel IV bedeutet,

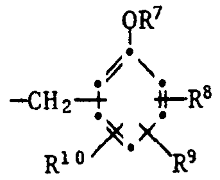

IV

und $R^{22}$ eine Gruppe -O($C_1$-$C_4$-Alkyl) oder eine Gruppe der Formel V

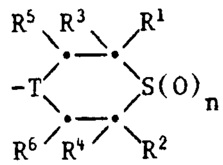

V

bedeutet.

**2.** Eine Verbindung gemäss Anspruch 1 der Formel I oder II, worin n 0 oder 2 ist,
$R^1$ und $R^2$ Wasserstoff oder Methyl bedeuten,
$R^3$ und $R^4$ unabhängig voneinander Methyl, Phenyl, Thienyl oder durch ein oder zwei $C_1$-$C_4$-Alkylgruppen, Cyclohexyl, Hydroxyl, $C_1$-$C_4$-Alkoxy oder Chlor substituiertes Phenyl bedeuten,
$R^5$ und $R^6$ unabhängig voneinander Wasserstoff, -COO($C_1$-$C_4$-Alkyl) oder -$COCH_3$ bedeuten,
$R^7$ Wasserstoff, eine Gruppe -CO-$R^{11}$, -CO-COO($C_1$-$C_4$-Alkyl), -$Si(CH_3)_3$ oder

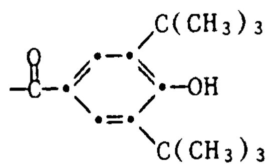

bedeutet,
$R^8$ und $R^9$ unabhängig voneinander $C_1$-$C_8$-Alkyl, $C_7$-$C_9$-Phenylalkyl, Cyclohexyl oder Phenyl bedeuten,
$R^{10}$ Wasserstoff, -$OR^7$ oder eine Gruppe der Formel III bedeutet,
worin M -S-, -$CH_2$-, -CH($C_1$-$C_4$-Alkyl) oder -$C(CH_3)_2$- ist,
$R^{11}$ $C_1$-$C_{12}$-Alkyl oder Phenyl bedeutet,
T eine der folgenden dreiwertigen Gruppen ist:
$>$CH-O-, $>$C=N-NH-,

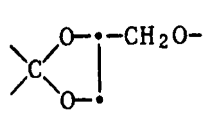 oder 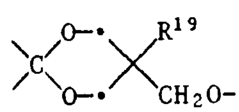 ,

worin $R^{19}$ $C_1$-$C_4$-Alkyl bedeutet,
und Z eine der folgenden zweiwertigen Gruppen ist:

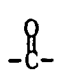, 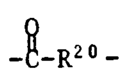, 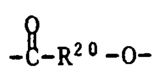 oder 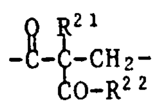 ,

deren Carbonylgruppe an T gebunden ist und worin
$R^{20}$ $C_1$-$C_{14}$-Alkylen ist,
$R^{21}$ $C_1$-$C_8$-Alkyl, Benzyl oder eine Gruppe der Formel IV ist und
$R^{22}$ eine Gruppe -O($C_1$-$C_4$-Alkyl) oder eine Gruppe der Formel V ist.

**3.** Eine Verbindung gemäss Anspruch 1, der Formel I und II, worin n 0 oder 2 ist,
$R^1$ und $R^2$ Wasserstoff oder Methyl bedeuten,
$R^3$ und $R^4$ Methyl, Phenyl, Thienyl oder durch $C_1$-$C_4$-Alkyl, $C_1$-$C_4$-Alkoxy, Hydroxyl oder Chlor substituiertes Phenyl bedeuten,
$R^5$ und $R^6$ Wasserstoff sind,
$R^7$ Wasserstoff oder eine Gruppe

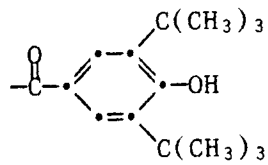

bedeutet,
$R^8$ und $R^9$ unabhängig voneinander Wasserstoff, $C_1$-$C_4$-Alkyl, Cyclohexyl oder Phenyl bedeuten,
$R^{10}$ Wasserstoff ist,
T eine der folgenden dreiwertigen Gruppen ist:
$>$CH-O-,

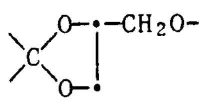 oder 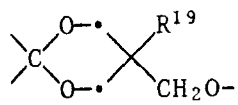 ,

worin $R^{19}$ $C_1$-$C_4$-Alkyl ist,
und Z eine der folgenden zweiwertigen Gruppen ist:

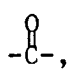, 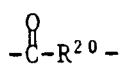 oder 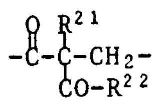 ,

deren Carbonylgruppe an T gebunden ist und worin
$R^{20}$ $C_1$-$C_8$-Alkylen bedeutet,
$R^{21}$ $C_1$-$C_8$-Alkyl, Benzyl oder eine Gruppe der Formel IV ist und
$R^{22}$ -O($C_1$-$C_4$-Alkyl) oder eine Gruppe der Formel V ist.

**4.** Eine Verbindung gemäss Anspruch 1 der Formel I oder II, worin $R^1$, $R^2$, $R^5$ und $R^6$ Wasserstoff sind und die übrigen Symbole die in Anspruch 1 gegebene Bedeutung haben.

**5.** Eine Verbindung gemäss Anspruch 1 der Formel I.

**6.** Eine Verbindung gemäss Anspruch 1 der Formel I oder II worin n 0 (null) ist.

**7.** Die Verbindung gemäss Anspruch 1, 3-[3-(3,5-Di-tert.butyl-4-hydroxyphenyl)-propionyloxymethyl]-3-ethyl-8,10,diphenyl-1,5-dioxa-9-thiaspiro[5.5]undecan.

**8.** Verwendung einer Verbindung der Formel I oder II gemäss Anspruch 1 als Stabilisator für farbphotographische Aufzeichnungsmaterialien.

**9.** Verwendung gemäss Anspruch 8 als Stabilisator für die Gelbschicht eines farbphotographischen Aufzeichnungsmaterials.

**10.** Farbphotographisches Aufzeichnungsmaterial enthaltend als Stabilisator in mindestens einer Schicht mindestens eine Verbindung der Formel I oder II gemäss Anspruch 1.

**11.** Farbphotographisches Material gemäss Anspruch 10, enthaltend in der Gelbschicht mindestens einen Stabilisator der Formel I oder II.

## Claims

**1.** A compound of the formula I or II

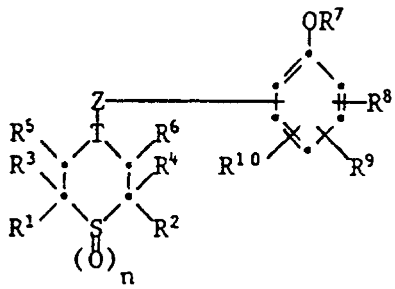

(I)

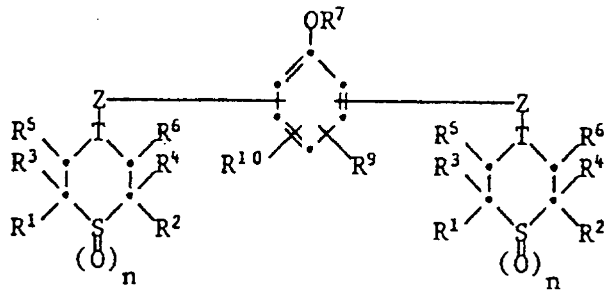

(II)

in which n is 0, 1 or 2, $R^1$ and $R^2$ independently of one another are hydrogen or methyl, $R^3$ and $R^4$ independently of one another are hydrogen, $C_1$-$C_4$alkyl, phenyl, thienyl or phenyl which is substituted by 1 or 2 $C_1$-$C_8$alkyl groups, cyclohexyl, phenyl, $C_7$-$C_9$phenylalkyl, hydroxyl, $C_1$-$C_{18}$alkoxy or halogen, $R^5$ and $R^6$ independently of one another are hydrogen, $C_1$-$C_4$alkyl, phenyl, -COO($C_1$-$C_{18}$alkyl), -CO-$CH_3$ or -CO-phenyl, $R^7$ is hydrogen, $C_1$-$C_8$alkyl or one of the groups -CO-$R^{11}$, -CO-COO($C_1$-$C_4$alkyl), -$SO_2$-$R^{12}$, -CON($R^{13}$)($R^{14}$), -Si($R^{15}$)($R^{16}$)($R^{17}$) or

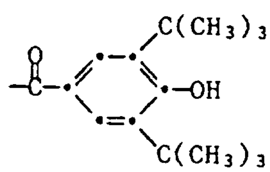

$R^8$ and $R^9$ independently of one another are hydrogen, $C_1$-$C_{12}$alkyl, $C_7$-$C_9$phenylalkyl, $C_5$-$C_8$cycloalkyl or phenyl, $R^{10}$ is hydrogen, -$OR^7$ or a group of the formula III

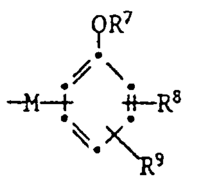

III

in which M is a direct bond, -O-, -S-, -S-S-, -$CH_2$-, -CH($C_1$-$C_8$alkyl)- or - C($CH_3$)$_2$-, $R^{11}$ is $C_1$-$C_{20}$alkyl, $C_3$-$C_{20}$alkenyl, $C_5$-$C_{12}$cycloalkyl, $C_7$-$C_{13}$phenylalkyl or $C_6$-$C_{10}$aryl, $R^{12}$ is $C_1$-$C_{12}$alkyl, $C_6$-$C_{10}$aryl or $C_7$-$C_{24}$alkylaryl, $R^{13}$ is hydrogen, $C_1$-$C_{12}$alkyl or cyclohexyl, $R^{14}$ is $C_1$-$C_{12}$alkyl, $C_6$-$C_{10}$aryl, $C_1$-$C_{12}$alkyl-substituted $C_6$-$C_{10}$aryl or cyclohexyl, or $R^{13}$ and $R^{14}$, togetner with the N atom, form a 5 - or 6-membered saturated heterocyclic ring, $R^{15}$, $R^{16}$ and $R^{17}$ independently of one another are $C_1$-$C_{12}$alkyl, $C_3$-$C_{12}$alkenyl, phenyl, cyclohexyl or benzyl, T is a trivalent group which completes the ring to give a thiane ring and is one of the following groups:

>CH-O-, >C=N-NH-,

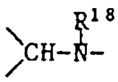 , 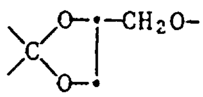 or 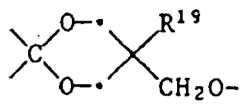 ,

in which $R^{18}$ is hydrogen, $C_1$-$C_{12}$alkyl, benzyl, cyclohexyl or phenyl and $R^{19}$ is hydrogen or $C_1$-$C_4$alkyl, Z is a divalent bonding member between T and the phenol radical and is one of the following groups:

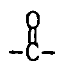 , 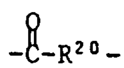 , 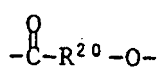 or 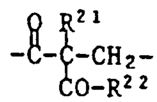 ,

the carbonyl group of which is bonded to T and in which $R^{20}$ is $C_1$-$C_{14}$alkylene, $R^{21}$ is hydrogen, $C_1$-$C_{12}$alkyl, phenyl, $C_7$-$C_9$phenylalkyl or a group of the formula IV

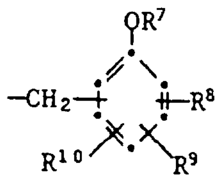

IV

and $R^{22}$ is a group -O($C_1$-$C_4$alkyl) or a group of the formula V

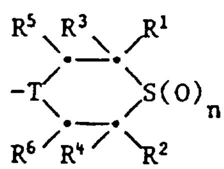

V

**2.** A compound according to claim 1 of the formula I or II, in which n is 0 or 2, $R^1$ and $R^2$ are hydrogen or methyl, $R^3$ and $R^4$ independently of one another are methyl, phenyl, thienyl or phenyl which is substituted by one or two $C_1$-$C_4$alkyl groups, cyclohexyl, hydroxyl, $C_1$-$C_4$alkoxy or chlorine, $R^5$ and $R^6$ independently of one another are hydrogen, -COO($C_1$-$C_4$alkyl) or $-COCH_3$, $R^7$ is hydrogen or a group $-CO-R^{11}$, -CO-COO($C_1$-$C_4$alkyl), $-Si(CH_3)_3$ or

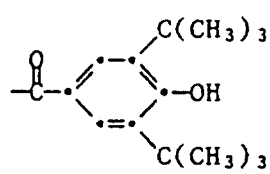

$R^8$ and $R^9$ independently of one another are $C_1$-$C_8$alkyl, $C_7$-$C_9$phenylalkyl, cyclohexyl or phenyl, $R^{10}$ is hdyrogen, $-OR^7$ or a group of the formula III, in which M is -S-, $-CH_2-$, -CH($C_1$-$C_4$alkyl) or $-C(CH_3)_2-$, $R^{11}$ is $C_1$-$C_{12}$alkyl or phenyl, T is one of the following trivalent groups:

>CH-O-, >C=N-NH-,

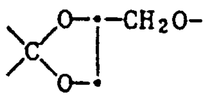 or 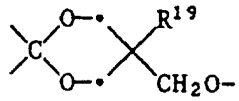 ,

in which $R^{19}$ is $C_1$-$C_4$alkyl, and Z is one of the following divalent groups:

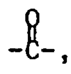, 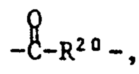, 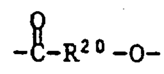 or 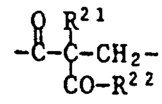 ,

the carbonyl group of which is bonded to T and in which $R^{20}$ is $C_1$-$C_{14}$alkylene, $R^{21}$ is $C_1$-$C_8$alkyl, benzyl or a group of the formula IV and $R^{22}$ is a group -O($C_1$-$C_4$alkyl) or a group of the formula V.

**3.** A compound according to claim 1 of the formula I or II, in which n is 0 or 2, $R^1$ and $R^2$ are hydrogen or methyl, $R^3$ and $R^4$ are methyl, phenyl, thienyl or phenyl which is substituted $C_1$-$C_4$alkyl, $C_1$-$C_4$alkoxy, hydroxyl or chlorine, $R^5$ and $R^6$ are hydrogen, $R^7$ is hydrogen or a group

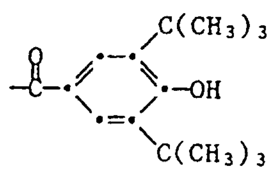 ,

$R^8$ and $R^9$ independently of one another are hydrogen, $C_1$-$C_4$alkyl, cyclohexyl or phenyl, $R^{10}$ is hydrogen, T is one of the following trivalent groups:
>CH-O-,

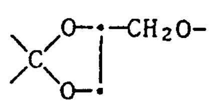 or 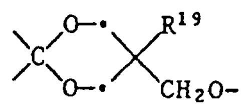 ,

in which $R^{19}$ is $C_1$-$C_4$alkyl, and Z is one of the following divalent groups:

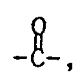, 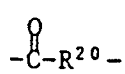 or 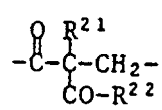 ,

the carbonyl group of which is bonded to T and in which $R^{20}$ is $C_1$-$C_8$alkylene, $R^{21}$ is $C_1$-$C_8$alkyl, benzyl or a group of the formula IV and $R^{22}$ is -O($C_1$-$C_4$alkyl) or a group of the formula V.

**4.** A compound according to claim 1 of the formula I or II, in which $R^1$, $R^2$, $R^5$ and $R^6$ are hydrogen and the other symbols are as defined in claim 1.

**5.** A compound according to claim 1 of the formula I.

**6.** A compound according to claim 1 of the formula I or II, in which n is 0 (zero).

**7.** The compound according to claim 1, 3-[3-(3,5-di-tert-butyl-4-hydroxyphenyl)-propionyloxymethyl]-3-ethyl-8,10-diphenyl-1,5-dioxa-9-thiaspiro[5.5]undecane.

**8.** The use of a compound of the formula I or II according to claim 1 as a stabilizer for colour photography recording materials.

9. The use according to claim 8 as a stabilizer for the yellow layer of a colour photography recording material.

10. A colour photography recording material containing at least one compound of the formula I or II according to claim 1 as the stabilizer in at least one layer.

11. A colour photography material according to claim 10 containing at least one stabilizer of the formula I or II in the yellow layer.

## Revendications

1. Un composé de formule I ou II,

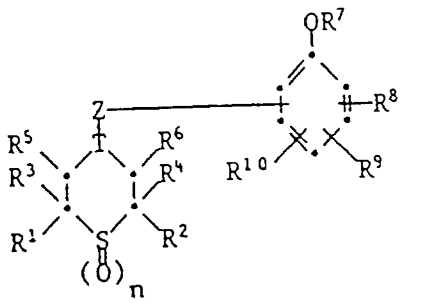

(I)

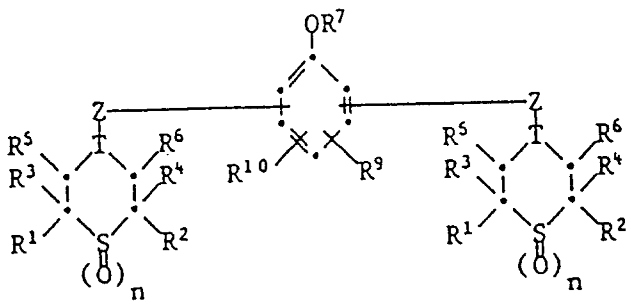

(II)

dans lesquelles n est 0, 1 ou 2,
$R^1$ et $R^2$ signifient indépendamment l'un de l'autre l'hydrogène ou un méthyle,
$R^3$ et $R^4$ signifient indépendamment l'un de l'autre l'hydrogène, un alkyle en $C_1$-$C_4$, un phényle, un thiényle ou un phényle substitué par 1 ou 2 groupes alkyle en $C_1$-$C_8$,cyclohecyle, phényle, phénylalkyle en $C_7$-$C_9$, hydroxyle, alcoxy en $C_{18}$ ou halogène,
$R^5$ et $R^6$ signifient indépendamment l'un de l'autre l'hydrogène, un alkyle en $C_1$-$C_4$, un phényle, -COO-(alkyl en $C_1$-$C_{18}$), -CO-$CH_3$ ou -CO-phényle,
$R^7$ signifie l'hydrogène, un alkyle en $C_1$-$C_8$, ou un des groupes -CO-$R^{11}$, -CO-COO(alkyl en $C_1$-$C_4$), -$SO_2$-$R^{12}$, -CON($R^{13}$)($R^{14}$), -Si($R^{15}$)($R^{16}$)($R^{17}$) ou

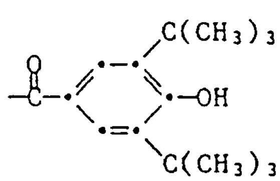

$R^8$ et $R^9$ signifient indépendamment l'un de l'autre l'hydrogène, un alkyle en $C_1$-$C_{12}$, un phénylakyle en $C_7$-$C_9$, un cycloalkyle en $C_5$-$C_8$ ou un phényle,
$R^{10}$ signifie l'hydrogène, -$OR^7$ ou un groupe de formule III

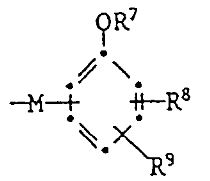

III

dans lequel M signifie une liaison directe, -O-, -S-, -S-S-, $-CH_2-$, -CH(alkyl en $C_1$-$C_8$) ou $-C(CH_3)_2-$,
$R^{11}$ signifie un alkyle en $C_1$-$C_{20}$, un alcényle en $C_3$-$C_{20}$, un cycloalkyle en $C_5$-$C_{12}$, un phénylalkyle en $C_7$-$C_{13}$ ou un aryle en $C_6$-$C_{10}$,
$R^{12}$ signifie un alkyle en $C_1$-$C_{12}$, un aryle en $C_6$-$C_{10}$ ou un alkylaryle en $C_7$-$C_{24}$,
$R^{13}$ signifie l'hydrogène, un alkyle en $C_1$-$C_{12}$ ou un cyclohexyle,
$R^{14}$ signifie un alkyle en $C_1$-$C_{12}$, un aryle en $C_6$-$C_{10}$, un cyclohexyle ou un aryle en $C_6$-$C_{10}$ substitue par un alkyle en $C_1$-$C_{12}$, ou $R^{13}$ et $R^{14}$ ensemble avec l'atome de N forment un cycle hétérocyclique sature à 5 ou 6 chaînons,
$R^{15}$, $R^{16}$ et $R^{17}$ indépendamment l'un de l'autre signifient un alkyle en $C_1$-$C_{12}$, un alcényle en $C_3$-$C_{12}$, un phényle, un cyclohexyle ou un benzyle.
T est un groupe trifonctionnel qui complète le cycle en un cycle thianne et est l'un des groupes suivants :
$>$CH-O-, $>$C=N-NH-,

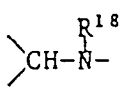 , 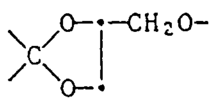 ou 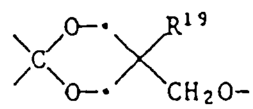 ,

dans lequel $R^{18}$ est l'hydrogène, un alkyle en $C_1$-$C_{12}$, un benzyle, un cyclohexyle ou un phényle et $R^{19}$ est l'hydrogène ou un alkyle en $C_1$-$C_4$,
Z est un groupe bifonctionnel de liaison entre T et le radical phénol et est un des groupes suivants :

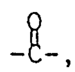 , 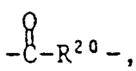 , 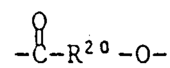 ou 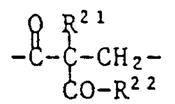 ,

dont le groupe carbonyle est lié à T et dans lesquels
$R^{20}$ signifie un alkylène en $C_1$-$C_{14}$,
$R^{21}$ signifie l'hydrogène, un alkyle en $C_1$-$C_{12}$, un phényle, un phénylalkyle en $C_7$-$C_9$ ou un groupe de formule IV

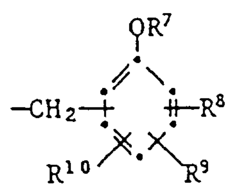

IV

et $R^{22}$ signifie un groupe -O(alkyl en $C_1$-$C_4$) ou un groupe de formule V

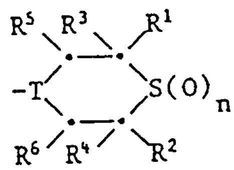

V

2. Un composé selon la revendication 1 de formule I ou II dans lequel n est 0 ou 2, $R^1$ et $R^2$ signifient l'hydrogène ou le méthyle, $R^3$ et $R^4$ signifient indépendamment l'un de l'autre un méthyle, un phényle, un thiényle ou un phényle substitué par un ou deux groupes alkyle en $C_1$-$C_4$, cyclohexyle, hydroxyle, alcoxy en $C_1$-$C_4$ ou le chlore, $R^5$ et $R^6$ signifient indépendamment l'un de l'autre l'hydrogène, -COO(alkyl en $C_1$-$C_4$) ou $-COCH_3$, $R^7$ signifie l'hydrogène, un groupe $-CO-R^{11}$, -CO-COO(alkyl en $C_1$-$C_4$), $-Si(CH_3)_3$ ou

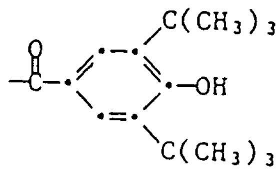

$R^8$ et $R^9$ signifient indépendamment l'un de l'autre un alkyle en $C_1$-$C_8$, un phénylalkyle en $C_7$-$C_9$, un cyclohexyle ou un phényle, $R^{10}$ signifie l'hydrogène, $-OR^7$ ou un groupe de formule III dans lequel M est -S-, $-CH_2-$, -CH(alkyl en $C_1$-$C_4$) ou $-C(CH_3)_2-$, $R^{11}$ signifie un alkyle en $C_1$-$C_{12}$ ou un phényle, T est un des groupes trifonctionnels suivants :
$>$CH-O-, $>$C=N-NH-,

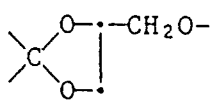 ou 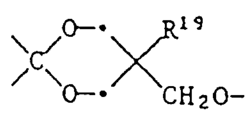 ,

où $R^{19}$ signifie un alkyle en $C_1$-$C_4$, et Z est un des groupes bifonctionnels suivants :

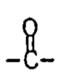, 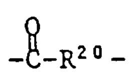, 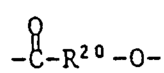 ou 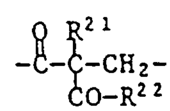 ,

dont le groupe carbonyle est lié à T et dans lesquels $R^{20}$ est un alkyle en $C_1$-$C_{14}$, $R^{21}$ est un alkyle en $C_1$-$C_8$, un benzyle ou un groupe de formule IV et $R^{22}$ est un groupe -O(alkyl en $C_1$-$C_4$) ou un groupe de formule V.

3. Un composé selon la revendication 1 de formule I et II où n est 0 ou 2, $R^1$ et $R^2$ signifient l'hydrogène ou un méthyle, $R^3$ et $R^4$ signifient un méthyle, un phényle, un thiényle ou un phényle substitué par des substituants choisis parmi les suivants : alkyle en $C_1$-$C_4$, alcoxy en $C_1$-$C_4$, hydroxyle ou chlore, $R^5$ et $R^6$ sont l'hydrogène, $R^7$ signifie l'hydrogène ou un groupe

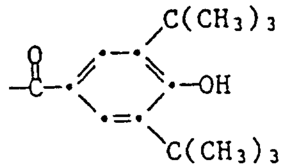

$R^8$ et $R^9$ indépendamment l'un de l'autre signifient l'hydrogène, un alkyle en $C_1$-$C_4$, un cyclohexyle ou un phényle,
$R^{10}$ est l'hydrogène,
T est un des groupes trifonctionnels suivants :
$>$CH-O-,

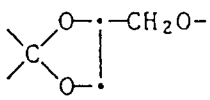 ou 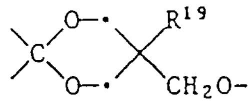 ,

où $R^{19}$ est un alkyle en $C_1$-$C_4$,
et Z est l'un des groupes bifonctionnels suivants :

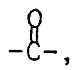, 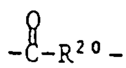 ou 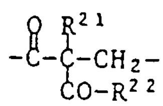 ,

dont le groupe carbonyle est lié à T et dans lesquels
$R^{20}$ signifie un alkylène en $C_1$-$C_8$,
$R^{21}$ est un alkyle en $C_1$-$C_8$, un benzyle ou un groupe de formule IV et
$R^{22}$ est -O(alkyl en $C_1$-$C_4$) ou un groupe de formule V.

**4.** Un composé selon la revendication 1 de formule I ou II dans lesquelles $R^1$, $R^2$, $R^5$ et $R^6$ sont l'hydrogène et les autres symboles ont la signification donnée dans la revendication 1.

**5.** Un composé selon la revendication 1 de formule I.

**6.** Un composé selon la revendication 1 de formule I ou II dans lequel n est 0 (zéro).

**7.** Le composé selon la revendication 1, le 3-[3-(3,5-di-tert.butyl-4-hydroxyphényl)-propionyloxyméthyl]-3-éthyl-8,10,diphényl-1,5-dioxa-9-thiaspiro[5.5]undécane.

**8.** Utilisation d'un composé de formule I ou II selon la revendication 1 en tant que stabilisant pour matériaux d'enregistrement photographique en couleur.

**9.** Utilisation selon la revendication 8 en tant que stabilisant pour la couche jaune d'un matériau d'enregistrement photographique en couleurs.

**10.** Matériau d'enregistrement photographique en couleurs contenant en tant que stabilisant dans au moins une des couche au moins un composé de formule I ou II selon la revendication 1.

**11.** Matériau photographique en couleurs selon la revendication 10 contenant dans la couche jaune au moins un stabilisant de formule I ou II.